(19) Europäisches Patentamt
European Patent Office
Office européen des brevets

(11) Publication number: **0 263 071 B1**

# (12) EUROPEAN PATENT SPECIFICATION

(45) Date of publication of patent specification: **12.08.92**

(21) Application number: **87810560.0**

(22) Date of filing: **25.09.87**

(51) Int. Cl.⁵: **C07D 487/04**, C07D 487/08, C07D 471/14, C07D 487/14, C07D 495/14, C07D 491/147, C07D 498/14, C07H 17/02, A61K 31/495, A61K 31/70, //(C07D487/04,249:00,239:00), (C07D487/08,249:00,239:00), (C07D487/14,249:00,239:00, 209:00),(C07D471/14,249:00, 239:00,221:00),(C07D487/14, 249:00,239:00,235:00), (C07D487/14),C07D249:00

## (54) 2-Substituted-e-fused-[1,2,4]-triazolo[1,5-c] pyrimidines, pharmaceutical compositions, and uses thereof.

(30) Priority: **30.09.86 US 913173**
**27.02.87 US 20055**

(43) Date of publication of application:
**06.04.88 Bulletin 88/14**

(45) Publication of the grant of the patent:
**12.08.92 Bulletin 92/33**

(84) Designated Contracting States:
**AT BE CH DE ES FR GB GR IT LI LU NL SE**

(56) References cited:
**EP-A- 0 181 282**
**EP-A- 0 217 748**

(73) Proprietor: **CIBA-GEIGY AG**
**Klybeckstrasse 141**
**CH-4002 Basel(CH)**

(72) Inventor: **Francis, John E.**
**15 Wexford Way**
**Basking Ridge, N.J. 07920(US)**
Inventor: **Gelotte, Karl O.**
**106 Stanie Brae Drive**
**Watchburg, N.J. 07060(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 263 071 B1

Alfred Burger, Medicinal Chemistry, 3rd Edition, 1970, pages 74-77

## Description

The invention relates to new 2-substituted e-fused-[1,2,4]triazolo[1,5-c]pyrimidines of the formula

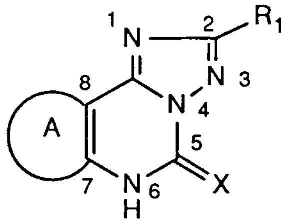

(I),

wherein

a) X is oxygen, $R_1$ is 2-fluorophenyl and ring A is $\Delta^3$-piperideine substituted at nitrogen by methoxycarbonylmethyl, 3-picolyl, 2-picolyl, 4-picolyl, phenylsulfonyl, carboxymethyl, carbamoylmethyl, carbamoyl, cyanomethyl, tetrazolylmethyl, (+)-α-methylbenzyl or (-)-α-methylbenzyl; or

b) X is oxygen, $R_1$ is 2-chlorophenyl, 2-pyridyl or 2-pyrrolyl and ring A is N-benzyl-$\Delta^3$-piperideine ; or

c) X is imino, $R_1$ is 2-furyl and ring A is cyclopentene;

a tautomer thereof or a pharmaceutically acceptable salt of said compound or tautomer;
any of these compounds for use in the treatment of anxiety or asthma or as adenosine antagonists, pharmaceutical compositions containing an effective amount of any of these compounds and a pharmaceutically acceptable currier, and processes for their preparation.

Also within the scope of the invention are the tautomeric forms of these compounds within formula I; typical of these tautomers are the structures below

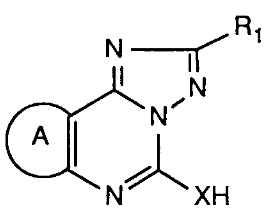

(Ib)

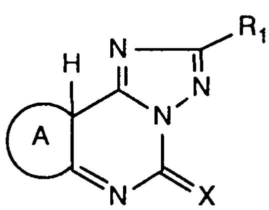

(Ic)

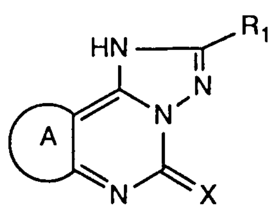

(Id)

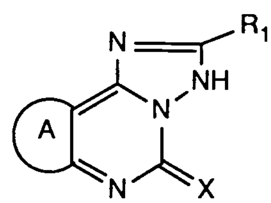

(Ie)

which may arise spontaneously. Other tautomers which may spontaneously form (depending on the particular A ring) are also within the scope of the invention.

Triazolo[1,5-c]pyrimidines have been described in a number of references. US Patents 3,045,015 and 3,053,844 disclose primarily bicyclic compounds having an unsubstituted amino group in the two-position of the triazolo pyrimidine ring. However, some tricyclic rings are generically set forth such as those that have a cyclohex-1-ene ring fused to the [e] face of the pyrimidine ring. These compounds are claimed as bronchodilators, respiratory stimulants, and antiarthritic agents. In addition, antibacterial, sedative, and hypotensive properties are disclosed. In J. Med. Chem. 17, 645-8 (1974), Novinson concludes that the compounds in US Patent 3,045,015 are active because they inhibit CAMP phosphodiesterase.

Shishoo, in J. Heterocyclic Chem. 18, 43-6 (1981), reports on the synthesis of angular tricyclics which are triazolopyrimidines having a heterocyclic fused to the [e] face of the pyrimidine ring. These are unsubstituted at the 2-position of the triazolopyrimidine ring system and have hydrogen, alkyl, aryl, or arylalkyl in place of the invention X.

Other heterocyclic rings fused to the [e] face of the [1,2,4]triazolo[1,5-c]pyrimidine ring system are mentioned in Huang et al, Chem. Pharm. Bull. 22 (8) 1938-9 (1974); Huang et al, Tetrahedron 31, 1363-7 (1975); Leonard and Wiemar; J.O.C. 39, 3438-40 (1974); Temple et al, U.O.C. 30, 3601-3 (1965); Sauter and Stanetty, Monatsh. Chem. 106, 1111-6 (1975); Brown and Shinozuka, Australian J. Chem. 34, 189-194 (1981); Bhat, Schram and Townsend, C.A. 95, 98200z (1981); Bhat and Townsend, J.C.S. Perkin I, 1981, 2387-2393; Schneller and Clough, J. Heterocyclic Chem. 1974, 975-7; Sangapure and Agasimundin, Indian J. Chem. B, 1980, 115-117; Saikachi, Matsuo and Matsuda, Yakugaku Zasshi 1969, 1434-9; and Petric, Tisler and Stanovnik, Monatsh. Chem. 114, 615-624 (1983). None of these compounds possess a carbonyl

or imino at the triazolopyrimidine position 5. Further, only Saikachi et al mentions any compound having an aryl group at the 2-position of triazolopyrimidine. No biological properties are indicated.

Triazolopyrimidines having a phenyl ring fused at the [e] face, instead of the present invention ring A, are mentioned in US Patents 4,463,007 and 4,053,600. Analogous pyrazolo pyrimidines are mentioned in US Patents 4,112,096; 4,112,098; 4,128,644; and 4,164,578. US Patent 4,585,772 discloses imidazopyrimidines or tetrahydropyrimidopyrimidines having a phenyl ring or a 6-membered heterocyclic having 5 carbons and nitrogen or 4 carbons and 2 nitrogens fused thereto. US Patents 4,087,423 and 4,124,764 disclose pyrazolotriazolo[4,3-c]pyrimidines. US Patents 4,479,955; 4,560,689; and 4,602,014 relate to pyrazolo pyridines having a partially saturated carbocyclic or heterocyclic ring fused to the pyridine ring.

EP-A-0 181 282 as regular prior art discloses compounds of the formula I wherein ring A is a planar, unsaturated ring. The physiological activity of the compounds of the present invention that have non-planar piperideine and cyclopentene ring systems and therefore are no classical bioisosteres could not be predicted on the basis of EP-A-0 181 282.

EP-A-0 217 748 is an intermediate document applicable under Article 54(3) with respect to novelty. It discloses in a generic way compounds of the formula I wherein ring A can be non-planar but does not disclose the specific compounds of the present invention.

Of all the compounds set out above, the only triazolopyrimidines having indications of (beneficial) biological activity have the 2-position of the triazolopyrimidine ring system substituted with hydrogen, an unsubstituted amino group, a carbonyl group, or lower alkyl. Therefore, it would be expected that one of these groups or a closely related one would be indespensable for useful biological properties to be present. Quite surprisingly, it has now been found that replacement of these groups with the mentioned substituents yields biologically active compounds as well. The new, useful compounds of the invention, especially when X is oxygen, affect benzodiazepine receptors, and, particularly when X is NH, affect adenosine receptors. Those compounds wherein X is S are primarily intermediates in the production of the compounds when X is oxygen or NR.

The benzodiazepine receptor (BR) agonists find utility as anxiolytics, CNS depressants, and anticonvulsants. BR inverse agonists (previously included with antagonists) elicit a response from the receptor, but opposite that which would result from an agonist, and as such find utility as anorectics, CNS stimulating agents, agents to increase cognitive ability, and to counteract the effects of benzodiazepines. True benzodiazepine antagonists merely block the receptor from benzodiazepines without eliciting a response there from. They are primarily useful in countering the effects of benzodiazepine. To the extent that the BR agonists elicit less of a response than benzodiazepines, they can also be used to counteract the effects of a benzodiazepine; however, due to the elicting of an agonistic response from the receptor, it will necessarily be less efficacious than a similarly bound antagonist.

Adenosine agonists are primarily useful as antihypertensives. Adenosine antagonists find their primary utility as anti-asthmatic agents and in the treatment of bradyarrhythmias associated with clinical conditions such as myocardiac infarction ans sleep apnea.

As used within this specification:

"Lower alkyl" means $C_1$-$C_7$, preferably $C_1$-$C_5$, more preferably $C_1C_4$; alkyl.

"Lower alkoxy" means $C_1$-$C_7$, preferably $C_2$-$C_5$, more preferably $C_2$-$C_4$ alkoxy.

"Lower alkenyl" means $C_2$-$C_7$, preferably $C_2$-$C_5$, more preferably $C_2$-$C_4$ alkenyl.

"Lower alkinyl" means $C_2$-$C_7$, preferably $C_2$-$C_5$, more preferably $C_2$-$C_4$ alkynyl.

Halogen and halo means fluorine, chlorine, and bromine. Unless apparent otherwise, reference made to "compounds" will also include the tautomers thereof. For purposes of consistency, when referring to the triazolopyrimidine bicyclic structure, the numbering system shown in structure I above will be used.

Preferred compounds for affecting the BR are those in which X is oxygen. For affecting the adenosine receptor (AR), compounds wherein X is NH are preferred.

Especially preferred compounds are those mentioned in the Examples.

Some of the compounds of the present invention can form acid addition salts, preferably pharmaceutically acceptable salts. Salts which are not pharmaceutically acceptable are suitable as intermediates for the preparation of the pharmaceutically acceptable salts or in the process of converting one compound of formula I into another of formula I. The acid addition salts are inorganic, exemplified by halide salts (such as chlorides), and sulfates, or organic which are typically examplified by sulfonated or carboxylated lower alkyl or aryl groups. Some suitable organic salts include acetate, methanesulfonate, toluenesulfonate, fumarate, cinnamate, and benzoate.

The Compounds of formula I wherein X is oxygen affect primarily the BR, while those wherein X is NH primarily influence the adenosine receptor. As such the oxo compounds (X = oxygen) exhibit anxiolytic and

anticonvulsant effects and antagonism of the effects of benzodiazepine drugs (such as carbamazepine). The imino compounds (X is NH) are adenosine antagonists and consequently are primarily useful as antiasthmatic agents and central nervous system stimulating agents (they enhance cognitive ability).

Hence, the compounds of the invention, especially those wherein X is oxygen, are useful in the treatment of nervous system disorders such as anxiety, convulsive conditions (i.e. epilepsy) and other disorders responsive to BR agonists or mixed agonist/antagonists.

The above effects are demonstrable in in vitro and in vivo tests using mammals, e.g. mice, rats, or monkeys, as test objects. The compounds can be administered enterally or parenterally, advantageously orally, subcutaneously, intravenously, or intraperitoneally in these tests, for example, in gelatin capsules or as aqueous solutions or suspensions. The dosage range for these tests is between about 0.1 and about 100 m/kg/day, preferably between about 0.5 and about 50 mg/kg/day, advantageously between about 1 and 25 mg/kg/day. For the in vitro tests, the applied dose range is between about $10^{-5}$ and about $10^{-10}$M concentration, preferably between about $10^{-7}$ and about $10^{-9}$M.

For treatment of the conditions set forth above in mammals, such as human beings, the compounds are administered orally, intraperitoneally, or by inhalation in doses in the range of 0.01 mg/kg to 500 mg/kg, preferably 0.1 mg/kg to 100 mg/kg, and most preferably about 10 mg/kg to about 30 mg/kg, body weight.

The benzodiazepine receptor binding properties indicative of the nervous system regulatory activity of said new compounds are determined in the receptor binding assay in vitro, e.g. similarly to that in Nature 266, 732 (1977) or Proc. Nat. Acad. Sci. USA 74, 3805 (1977). When tritiated flunitrazepam is used, the interaction of other drugs with said receptor can be readily assessed thus: Synaptosomal membranes from rat fore-brain are incubated at 0-5° for 30 minutes with 0.5 nM tritiated flunitrazepam and various concentrations of the test substance in a buffer medium maintained at pH 7.5. Solutions of the various concentrations of test substance are prepared by dilution of a 4.2 mM stock solution in dimethylacetamide-ethanol (1:10) with 50 mM pH 7.5 Tris-HCl buffer. The membranes, containing the receptors with various amounts of tritiated flunitrazepam, are filtered onto glass fiber filters, which are then analyzed in a liquid scintillation counter. The concentration of the compounds of this invention, required to inhibit the specific binding of 0.5 nM of tritiated flunitrazepam by 50 %, i.e. the $IC_{50}$, is determined graphically.

In vivo benzodiazepine receptor binding is determined essentially as described in Eur. J. Pharmacol. 48, 213 (1978) and Nature 275, 551 (1978).

Test compounds in a corn starch vehicle are administered orally or intraperitoneally to mice or rats.

Thirty minutes later, $^3$H-flunitrazepam (2 nmoles/Kg in saline) is injected into the tail vein, and the animals are sacrificed 20 minutes after injection of the flunitrazpam. The brains are then assayed by determining radioactivity in a liquid scintillation counter for binding of the radioligand to the receptors. A decrease in the binding of $^3$H-flunitrazepam in the drug-treated animals (as compared with the binding observed in animals treated with vehicle alone) is indicative of benzodiazepine receptor binding by the test compound.

Anxiolytic effects are observed, for example, according to the Cook-Davidson conflict procedure, using male Wistar rats which are maintained at 80 % of normal body weight by dietary-, but not water-restriction. They are trained to press a lever within a conditioning chamber, also containing a liquid dipper, a house light, a speaker and a grid-floor. The grids are conected to an electrical shock source and the chamber is situated in a sound-attenuated room in which a white noise-source is activated during testing, in order to mask any extraneous auditory cues. Each session of 47 minutes duration consists of two alternating schedules. The first is a Variable Interval (VI) schedule of 30 seconds, lasting for 5 minutes, during which a sweetened, condensed milk reinforcement is delivered following the first lever-press after an average of 30 seconds have elapsed, and a drug-induced decrement of this performance is taken as an indication of neurological deficit. Immediately following the VI-schedule both a 1000 Hz tone and light-cue are activated, indicating the commencement of the second Fixed Ratio (FR) schedule, lasting for 2 minutes, wherein the milk reinforcement is delivered concomitant with an electric foot shock immediately following he tenth response, thereby establishing a conflict situation. The intensity of said shock ranges between 1.0-2.5 mA, varying with each animal, in order to adjust them to about 25-100 responses during this schedule over the entire session. A drug-induced enhancement of performance during the FR-schedule is taken as indication of antianxiety effects. This increased performance is measured by the increased number of electric foot shocks taken during six FR sessions lasting 2 minutes each.

Anticonvulsant effects are observed, for example in the standard Metrazole (pentylenetetrazole) and maximal electroshock tests for assessing anticonvulsant activity, e.g. orally in the rat.

Male Wister rats (130-175 g) are fasted for 18 hours but allowed water as desired prior to testing. The test compound is administered in a cornstarch vehicle by oral intubation in a volume of 10 ml/kg of body weight. One hour after administration of the test compound the animals are administered intravenously

(caudal vein) a dose of 24 mg/Kg of Metrazole in water in a volume of 2.5 ml/Kg of body weight. The rats are immediately placed in plexiglass cylinders and observed for clonic seizures of at least 5 seconds durations during the next 60 seconds. The $ED_{50}$ is the dose at which half the animals are protected from Metrazole induced clonic seizures during the observation periods.

These and other methods are detailed more fully in Woods, J. Pharmacology and Experimental Therapeutics, Vol. 231, No. 3, 572-576 (1984).

The compound of the invention where X is imino acts as adenosine antagonist. Adenosine antagonism is assessed by determination of inhibition of adenosine activation of adenylate cyclase in vesicular preparations from guinea pig brains essentially as described in J. Neurochem. 22, 1031 (1974) and J. Neurochem. 38, 1437 (1982).

The compounds of formula I (their tautomers and pharmaceutically acceptable salts) are formulated into pharmaceutical compositions comprising an effective amount of the triazolopyrimidine compounds of formula I or a pharmaceutically acceptable salt thereof in combination with conventional excipients or carriers suitable for either enteral or parenteral, such as oral, bronchial, rectal or intravenous, administration. Preferred are tablets, dragees and gelatin capsules comprising the active ingredient together with A) diluents, e.g., lactose, dextrose, sucrose, mannitol, sorbitol, cellulose, calcium phosphates and/or glycine, b) lubricants, e.g. silica, talcum, stearic acid, its magnesium or calcium salt and/or polyethyleneglycol; for tablets also, c) binders, e.g. magnesium aluminium silicate, starch paste, gelatin, tragacanth, methylcellulose, sodium carboxymethylcellulose and/or polyvinylpyrrolidone; if desired, d) disintegrants, e.g. starches, agar alginic acid or its sodium salt, or effervescent mixtures and/or, e) absorbents, colorants, flavors and sweeteners. Dragee or tablet cores may be provided with suitable coatings, which may be resistant to gastric juices. Coating solutions are, for example, concentrated aqueous sugar solutions, which may contain gum arabic, polyvinylpyrrolidone, polyethylene glycol, talcum and/or titanium dioxide. Resistant coatings are obtained with lacquer solutions in organic solvents, such as shellac, acetylcellulose phthalate or hydroxypropylmethylcellulose phthalate in ethanol and the like. Dyestuffs or pigments may be added for identification of brand name and dose. Capsules are either made from hard gelatin, or they are soft, closed capsules made from gelatin and a softener, e.g., glycerin or sorbitol. The hard capsules contain either uncompressed powder mixtures, e.g. those mentioned under a) and b), or granulates similar to those used for tablets. In the soft capsules said active ingredients are preferably dissolved or suspended in suitable liquids, such as fatty oils, paraffins or polyethylene glycols. Suppositories are advantageously solid, fatty emulsions or suspensions, containing the active ingredient, for example, in natural or synthetic triglycerides, paraffins, waxes and/or polyethylene glycols.

Compositions for parenteral administration are preferably aqueous solutions or suspensions of said active substances, but also oily solutions or suspensions of said active substances, but also oily solutions or suspensions thereof, e.g., in natural or synthetic fatty oils, such as sesame oil or ethyl oleate, in suitable ampules.

Bronchial compositions are preferably aerosol sprays and may be administered from a dispenser such as is described in U.S. Patents 4,292,966, 4,174,712, and 4,137,914. The active ingredient is mixed with a propellant such as a hydrocarbon, chlorofluorocarbon mixture, or carbon dioxide.

The compositions may be sterilized and/or contain adjuvants, such as preserving, stabilizing, wetting or emulsifying agents, solution promoters, salts for regulating the osmotic pressure and/or buffers. They may also contain other therapeutically valuable substances, and are prepared according to conventional mixing, granulating or coating methods respectively. They may contain from about 10 to 95 %, preferably from about 20 to 70 % of the active ingredient. Individual unit dosages thereof for a mammal of about 50-70 Kg weight may contain preferably between about 10 and 200 mg, advantageously about 20 to 100 mg of said active ingredients.

Benzodiazepine antagonism is measured by the antagonism of the diazepam-induced rotorod deficit in the rat. Diazepam (30 mg/kg/ip) and test compound are administered 30 minutes and 1 hour, respectively, before the test.

In the maximal electroshock procedure for assessing anticonvulsant activity in rats, seizures are induced by applying 150 mA of electric current for 0.2 seconds through corneal electrodes two hours after oral administration of test compound. The $ED_{50}$ is the dose at which half the animals are protected from electroshock induced seizures during a 5 second observation period.

The pharmacological benzodiazepine agonist and/or antagonist profile of the compounds of the invention can also be determined by measuring their effect in a rat brain membrane preparation on the displacement of $^{3}H$-flunitrazepam in the presence or absence of gamma-aminobutyric acid (GABA), or on the enhancement of $^{3}H$-muscimol binding by etazolate, or on the binding by etazolate, or on the binding of $^{35}S$-butyl bicyclophosphorothionate (TBPS).

The compounds of the present invention can be prepared by methods known in the art. In addition, they can be prepared by the following methods.

The compounds of formula I can be prepared

a) by cyclizing a compound of the formula:

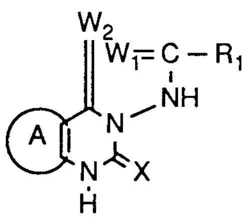

(II),

wherein $R_1$, X and ring A are as defined above, one of $W_1$ and $W_2$ is NH and the other one of $W_1$ and $W_2$ represents O or NH, by treatment with a base furnishing a nitrogen atom, preferably a tertiary amine such as triethylamine or piperidine. The compound of formula II is prepared by selective ring closure of a compound of formula

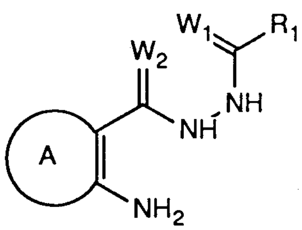

IX

wherein one of $W_1$ and $W_2$ is oxygen and the other is NH or wherein $W_1$ and $W_2$ are both NH. The compound of formula II wherein X is imino is prepared by selectively cyclizing a compound of the formula

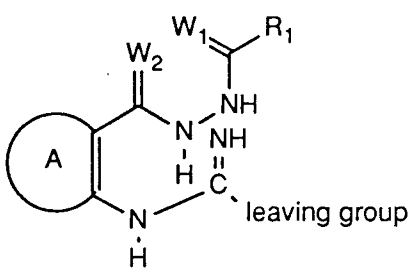

XVI

or

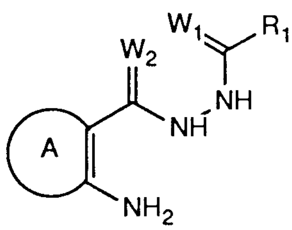

XVII

or

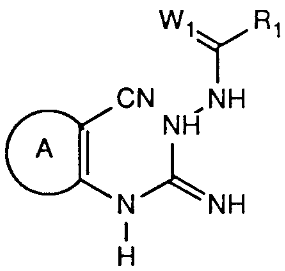

XVIII.

The compounds of formula XVI are prepared from those of formula XVII. The reaction of compounds of formula XVII to compounds of formula II wherein X is imino requires the presence of a reactive imino carbonic acid derivative, a cyanogen halide or cyanamide.

The compounds of formula XVIII are prepared from the reaction of a compound of the formula

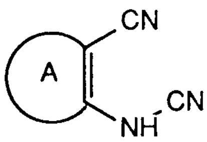

XIX

with a hydrazine of formula V given below. Ring closure is obtained preferably by treatment thereof with ammonia; or

b) to obtain a compound of the formula I, wherein X is oxygen, by cyclizing a compound of the formula

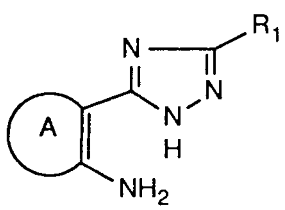

III

wherein $R_1$ and ring A are as defined above, with a reactive derivative of carbonic acid, which includes esters, amides, and anhydrides of carbonic acid. Included here are phosgene, diethylcarbonate, ethylcarbamate and trichloromethyl chloroformate.

The compounds of formula III can be prepared by treating a reactive intermediate of an alpha,beta-cyclic-beta-amino acrylic acid, such as tetrahydro isatoic anhydride, with a compound of the formula

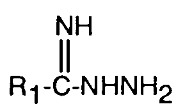

X

Alternatively, compounds of formula III can be prepared from compounds of the formula

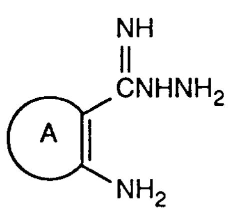

XI

and

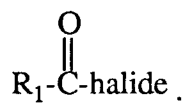

The alpha,beta-cyclic-beta-amino acrylic acid intermediates may also be of the formula

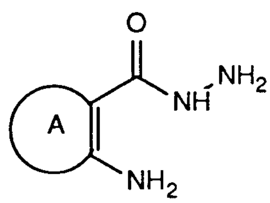

XII

which are reacted with

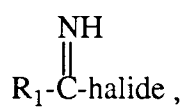

-ether, or-amine,

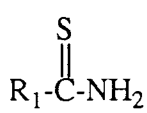 , 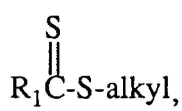

or

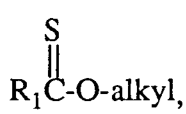

-halide or

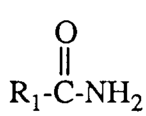

or halide, or the corresponding acid anhydride to yield a compound of formula III. Compounds of formula XII can be prepared from the corresponding acid of formula

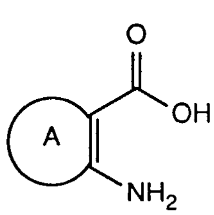

XIII

by esterification, followed by reacting with hydrazine.

Compounds of formula III can also be obtained by hydrolyzing the compounds of formula I by treatment there of with alkali metal hydroxide.

The compounds of formula III can further be prepared by selective ring closure in a compound of the formula IX given above wherein one of $W_1$ and $W_2$ is oxygen and the other is NH or wherein $W_1$ and $W_2$ are both NH; or

c) to obtain a compound of formula I, wherein X is oxygen, by reacting a compound of the formula:

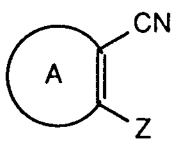

(IV)

wherein ring A is as defined above and Z is the radical of a carbonic acid derivative bonded via a nitrogen atom, preferably isocyanato,

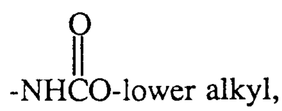

or

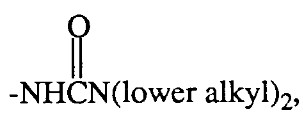

with a hydrazide of the formula

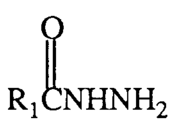

V.

The compounds of formula IV where Z is

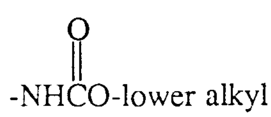

can be readily prepared by reacting

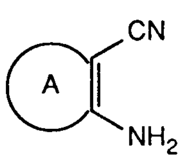

with 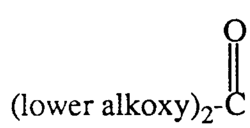 ;

or

d) to obtain a compound of formula I wherein x is oxygen, by treating a compound of the formula:

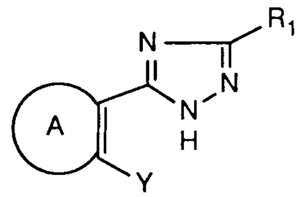

(VI)

wherein Y represents a group capable of being converted to the grouping -N=C=O by an oxidizing agent, with said oxidizing agent followed by ring closure, for example by treating a compound of the formula

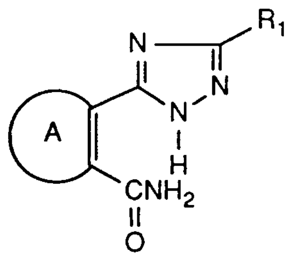

XIV

with an oxidizing agent followed by ring closure; or
e) to obtain the compound of formula I, wherein X is NH, by converting a compound of the formula:

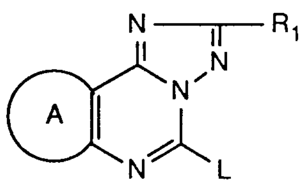

(VII)

wherein $R_1$ is 2-furyl, ring A is cyclopentane and L is selected from halogen, $C_1$-$C_7$-alkoxy, aryl-$C_1$-$C_7$-alkoxy, mercapto, $C_1$-$C_7$-alkylthio and isothiocyanato, by replacing the group L by the grouping NH; or
f) treating a compound of the formula:

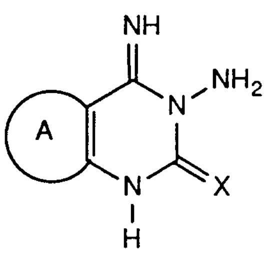

(VIII)

with a reactive derivative of a carboxylic acid of the formula $R_1$-COOH, which includes esters, amides, and anhydrides of carbonic acid. Included here are phosgene, diethylcarbonate, ethylcarbamate and trichloromethyl chloroformate.
g) to obtain the compound of formula I wherein X is imino, by treating a compound of formula I wherein X is NR, wherein R is lower alkyl, lower alkenyl or lower alkynyl, with aqueous acid; or
h) to obtain the compound of formula I wherein X is NH by i) reacting a compound of formula III, as defined in process (b) above, with cyanogen halide followed by cyclization or reacting a compound of formula III with cyanamide or a reactive imino carbonyl derivative; or
i) to obtain a compound of formula I wherein X is oxygen, by reacting a compound of formula IX with a

reactive derivative of carbonic acid and base; or

j) to obtain a compound of formula I wherein X is oxygen, by hydrolyzing a compound of the formula

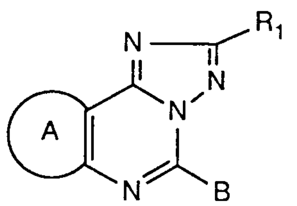

XV

wherein B is halo or lower alkoxy; or

k) to obtain the compound of formula I wherein X is imino, by doubly cyclizing a compound of formula XVI or XVII or XVIII, the reaction of the compound of formula XVII being in the presence of a cyanogen halide or cyanamide or a reactive imino carbonic acid derivative; or

l) to obtain the compound of formula I wherein X is imino, by cyclizing a compound of the formula

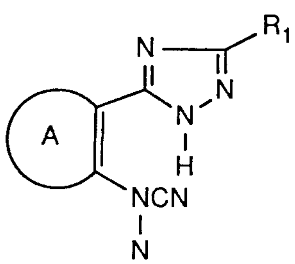

XX

which is prepared from a compound of formula III by reaction thereof with cyanogen halide; or

m) to obtain the compound of formula I wherein X is imino, by reacting a compound of the formula

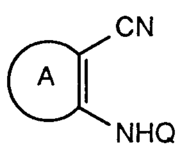

XXI

wherein Q is -CN or

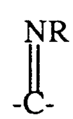

leaving group with a hydrazide of formula V; or

if desired by converting one claimed compound into another and/or in the case of compounds having salt forming groups by converting a salt into a claimed compound or into a different salt or a claimed compound into a salt, such as

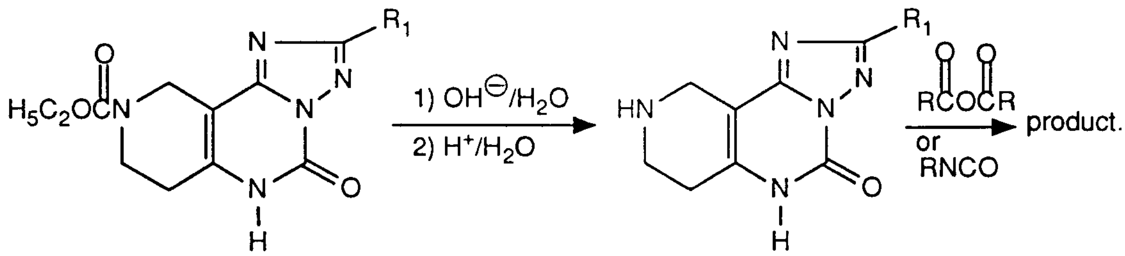


In a process variant (a), a compound of formula IX is converted to a compound of formula II wherein X is oxygen by reaction thereof with a reactive derivative of carbonic acid as that term is defined in process b. Use of the corresponding reactive derivative of imino carbonic acid (which includes cyanamide) will result in the corresponding compound of formula II wherein X is imino. The o-cyanimidobenzonitriles which replace instant ring A with a benzene ring are described by Wentrup in Tetrahedron, 27, 367 (1971) and by Bedford in J. Chem. Soc. 1633 (1959). The compound of formula XIX can also be converted directly into a compound of formula I by the described reaction, structure XVIII being an intermediate therein, as in process variant j.

In a process variant (b), a compound of formula IX is converted to a compound of formula III by reaction thereof with a base, preferably a tertiary amine or ammonia.

In process variant (c), the reaction takes place preferably in a solvent, such as an ether solvent, for example dioxane, or an alcohol solvent, for example 2-methoxyethanol, or a liquid amide such as dimethylacetamide.

When Z represents isocyanate or -NHC(=O)O lower alkyl, the reaction is conducted in the presence of a base such as a tertiary amine, for example N-methylmorpholine, triethylamine, and, especially, tripropylamine. The compounds having formula IV wherein Z represents isocyanato may be converted into the corresponding compounds wherein Z represents -NHC(=O)O-lower alkyl by treatment with a lower alkanol such as ethanol. Lower alkyl is preferably methyl or ethyl.

The compounds having formula IV wherein Z represents -NH(C=O)O-lower alkyl may also be formed by treating an alpha-beta-cyclic eneamino nitrile with lower alkyl chloroformate, for example ethyl chloroformate.

The compounds having formula IV wherein Z represents NHC(=O)-di-lower-alkyl may be formed by treating the appropriate O-isocyanatocyclic ene nitrile with a di-lower-alkylamine such as diethylamine.

The preferred solvents when Z represents isocyanato are ether solvents, especially dioxane. The preferred solvents when XH represents -NHC(=O)O-lower alkyl or NHC(=O)N-di-lower alkyl are alcohol solvents, especially 2-methoxyethanol. A preferred solvent in either case is a liquid amide, such as dimethyl acetamide. The reaction is preferably conducted at temperatures of 0 to 250 °C, preferably 20 to 150 °C.

The starting compounds of process c) of formula IV can be prepared by the Thorpe-Ziegler reaction as detailed in The Chemistry of Cyclic Eneaminonitriles and O-aminonitriles, ed. E.C. Taylor, Interscience (1970), New York,pp. 11 - 56.

In process variant (d), the oxidizing agent may be, for example, lead tetraacetate or a hypohalite. The hypohalite is preferably an alkali metal hypohalite such as sodium hypochlorite or sodium hypobromite. The amide function is believed to undergo the first stage of the Hofmann reaction (Ber. 14,2725 (1881)), forming the isocyanate which then reacts with the free NH of the triazole.

In process variant (h), the cyanogen halide is preferably cyanogen choride or cyanogen bromide, more preferably cyanogen bromide. The hydrohalide formed in the reaction may be neutralized with base, suitably triethylamine, pyridine, sodium hydride. The cyclization step is catalyzed by mineral acids or by bases, such as hydrohalides or trialkylamines, respectively.

In process (i), the reactive derivative of carbonic acid and the base are preferably those in variants a and b.

In process variant (j), a 5-halo, lower alkoxy or aryl lower alkoxy substituted e-fused [1,2,4]triazolo[1,5-c]-pyrimidine is hydrolyzed. The hydrolysis is preferably carried out with aqueous alkali.

The 5-halo compounds may be prepared be reacting a compound of formula I wherein X represents O with a reactive halide such as phosphoryl chloride or phosphorous pentachloride, most preferably phenylphosphonic dichloride with or without an inert solvent.

The 5-lower alkoxy or 5-aryl-lower alkoxy compounds may be prepared from the 5-halo compounds by treatment with the appropriate alcohol in the presence of a base, preferably a tertiary amine.

Having generally described the invention, a more complete understanding can be obtained by reference

to certain specific examples, which are provided herein for purposes of illustration only and do not limit the claims unless otherwise specified. Within the Examples, numbering of the ring systems is in accordance with the generally accepted rules of nomenclature.

Example 1:

The product of this Example is prepared by the method delineated in Example 11 of the European Patent EP 0 217 748. (The wording of Example 11 in EP 0 217 748 is: A mixture of the ethylcarbamate of N-benzyl-3-cyano-4-amino-$\Delta^3$-piperideine (10 g), p-fluorobenzhydrazide (4.41 g) and 1-methyl-2-pyrrolidine (80 ml) is stirred at reflux under nitrogen for 20 hours. It is evaporated at reduced pressure to remove most of the solvent, then diluted with isopropanol (100 ml) and stirred one-half hour. The precipitate product is collected, washed with isopropanol and dried. It is converted to the p-toluenesulphonate salt by treatment with an equimolar amount of p-toluenesulphonic acid in methanol. The salt is suspended in 2-methoxyethanol, filtered and converted back to the free base in dilute ammonium hydroxide, in 42 % yield. The pure 9-benzyl-2-(4-fluorophenyl)-7,8,9,10-tetrahydropyrido[3,4-e][1,2,4]triazolo[1,5-c]pyrimidine-5(6H)one melts in the range 252 to 256°.)

When 2-chlorobenzhydrazide is substituted for p-fluorobenzhydrazide in Example 11 of EP 0 217 748, 9-benzyl-2-(2-chlorophenyl)-7,8,9,10-tetrahydropyrido[3,4-e][1,2,4]-triazolo[1,5-c]-pyrimidin-5(6H)one is obtained. It is purified by conversion to the methanesulphonate salt in methanol, m.p. 295-296°.

Example 2:

When picolinic acid hydrazide is substituted for p-fluorobenzhydrazide in Example 11 of EP 0 217 748 (for text see Example 1), 9-benzyl-2-(2-pyridyl)-7,8,9,10-tetrahydropyrido[3,4-e][1,2,4]triazolo[1,5-c]-pyrimidin-5-(6H)one is obtained and purified as the methanesulphonate salt, mp. 290-290°.

Example 3:

When pyrrole-2-carboxylic acid hydrazide is substituted for p-fluorobenzhydrazide in Example 11 of EP 0 217 748 (for text see Example 1), 9-benzyl-2-(2-pyrrolyl)-7,8,9,10-tetrahydropyrido[3,4-e][1,2,4]-triazolo[1,5-c]pyrimidin-5(6H)one is obtained and purified by trituration in methanol, filtration and drying under reduced pressure. The free base, m.p. 297-298°, is obtained.

Example 4:

In a pressure vessel is placed 100 ml of ammonium hydroxide saturated with ammonia at -5° and 1.7 g 5-chloro-8,9-dihydro-2(2-furyl)-7H-cyclopenta[e][1,2,4]triazolo-[1,5-c]pyrimidine (prepared as described in Example 29 of the European Patent, EP 0 217 748) dissolved in 13 ml 1-methyl-2-pyrrolidone (13 ml). It is heated 5.5 h at an outside temperature of 150°, then allowed to cool and the solid collected, washed with water and air dried. After recrystallization from ethanol, 2-(2-furyl)-5-imino-5,6,8,9-tetrahydro-7H-cyclopenta-[e][1,2,4]triazolo[1,5-c]-pyrimidine mp. 255-260°, is obtained.

Example 5:

A mixture of 570 mg of the product of Example 15 of the European Patent EP 0 217 748, 2-(2-fluorophenyl)-7,8,9,10-tetrahydropyrido[3,4-e][1,2,4-triazolo[1,5-c]pyrimidin-5(6H)one, 8 ml ethyl bromoacetate and 15 ml dry dimethylformamide is stirred at 100° under nitrogen for 1.5 hours. It is poured into cold 8 % aqueous sodium bicarbonate solution and stirred vigorously to form crystals. The solid is collected, washed with water (2x20 ml), then ether (2x30 ml) and recrystallized from ethanol to afford 9-carbomethoxy-methyl-2-(2-fluorophenyl)-7,8,9,10-tetrahydropyrido[3,4-e][1,2,4]triazolo[1,5-c]pyrimidin-5(6H)one, m.p. 265-266°.

Example 6:

To a suspension of 280 mg of the product of Example 15 of the European Patent EP 0 217 748, 2-(2-fluorophenyl)-7,8,9,10-tetrahydropyrido[3,4-e][1,2,4-triazolo[1,5-c]-pyrimidin-5(6H)one, 197 mg of 3-picolyl chloride hydrochloride and 10 ml dry dimethylformamide under nitrogen is added 0.4 ml triethylamine under stirring and the whole stirred at ambient temperature over 94 hours. It is quenched in cold 5 % aqueous sodium hydroxide solution, whereupon crystals gradually form from solution. The product is washed with ice-water (2x5 ml), then ether (2x10 ml) and treated with twice the equimolar amount of methanesulphonic acid in methanol (10 ml). The resulting white crystalline product is collected, washed with ether (2x5 ml) and dried in vacuo to give pure 2-(2-fluorophenyl)-9-(3-picolyl)-7,8,9,10-tetrahydropyrido[3,4-e][1,2,4]triazolo[1,5-c]pyrimidin-5(6H)one.

Example 7 and 8:

By the method described in Example 6, 2(2-fluorophenyl)-9-(2-picolyl)-7,8,9,10-tetrahydropyrido[3,4-e]-[1,2,4]-triazolo[1,5-c]pyrimidin-5(6H)one dimethanesulphonate is obtained from 2-picolyl chloride hydrochloride and the corresponding 4-picolyl compound is prepared from 4-picolylchloride hydrochloride.

Example 9:

By a method similar to that described in Example 6, the product from Example 15 of the European Patent EP 0 217 748, 2-(2-fluorophenyl)-7,8,9,10-tetrahydropyrido[3,4-e][1,2,4-triazolo[1,5-c]-pyrimidin-5(6H)one, is reacted with benzene sulphonyl chloride to give 2-(2-fluorophenyl)-9-phenylsulphonyl-7,8,9,10-tetrahydropyrido[3,4-e][1,2,4]triazolo[1,5-c]-pyrimidin-5(6H)one.

Example 10:

To a suspension of 500 mg of the product of Example 5 in 20 ml ethanol is added 1 ml of 4N aqueous sodium hydroxide solution and the mixture is stirred at reflux conditions under nitrogen for 2 hours. The mixture is cooled, filtered and the filtrate neutralized with glacial acetic acid with ice cooling to produce the solid 9-carboxymethyl-2-(2-fluorophenyl)-7,8,9,10-tetrahydropyrido[3,4-e][1,2,4]triazolo[1,5-c] pyrimidin-5-(6H)one.

Example 11:

500 mg of the product of Example 10 are stirred in 5 ml thionyl chloride over 4 hours and then concentrated to dryness at reduced pressure. The residual material is treated with 50 ml of a cold saturated solution of ammonia in methanol and, after overnight stirring, the mixture is diluted with water and the product, 9-carbamoylmethyl-2-(2-fluorophenyl)-7,8,9,10-tetrahydropyrido[3,4-e][1,2,4]triazolo[1,5-c]pyrimidin-5(6H)one, collected and recrystallized from a mixture of dimethylacetamide and water.

Example 12:

To a solution of 570 mg of the product of Example 15 of the European Patent EP 0 217 748, 2-(2-fluorophenyl)-7,8,9,10-tetrahydropyrido[3,4-e][1,2,4-triazolo[1,5-c]-pyrimidin-5(6H)one, in 1:1 glacial acetic acid-water is added 324 mg potassium cyanate in 3 ml water under stirring at 35°. After reacting 2 hours, the mixture is heated briefly to 80°, cooled, diluted with water and the product, 9-carbamoyl-2-(2-fluorophenyl)-7,8,9,10-tetrahydropyrido[3,4-e][1,2,4]triazolo[1,5-c]pyrimidine-5(6H)one, collected, washed with water and purified by recrystallization from the mixture of dimethylacetamide with water.

Example 13:

A mixture of 570 mg of the product of Example 15 of the European Patent EP 0 217 748, 2-(2-fluorophenyl)-7,8,9,10-tetrahydropyrido[3,4-e][1,2,4-triazolo[1,5-c]-pyrimidin-5(6H)one, 360 mg bromoacetonitrile, 10 ml dimethylformamide and 0.5 ml triethylamine is stirred under nitrogen over 4 days. It is poured into 10 % aqueous sodium bicarbonate and stirred vigorously over 1/2 hour. The material is collected, washed with water, then ether and dried in vacuo to afford 9-cyanomethyl-2-(2-fluorophenyl)-7,8,9,10-tetrahydropyrido-[3,4-e]-[1,2,4]triazolo[1,5-c]pyrimidin-5(6H)one.

Example 14:

A mixture of 640 mg of the product of Example 13, 1.41 g sodium azide, 120 mg ammonium chloride and 10 ml dimethylformamide is stirred at 90° over 3 hours under nitrogen. The mixture is cooled and quenched in 40 ml ice-water, stirred 1/2 hour, and cautiously acidified with glacial acetic acid to afford the solid product 2-(2-fluorophenyl)-9-tetrazolylmethyl-7,8,9,10-tetrahydropyrido[3,4-e][1,2,4]triazolo[1,5-c]-pyrimidine-5(6H)one.

Examples 15 and 16:

The product of this Example is prepared by the method delineated in Example 8 of the European Patent EP 0 217 748. The wording of Example 8 in EP 0 217 748 is: A mixture of the ethylcarbamate of N-benzyl-3-cyano-4-amino-$\Delta^3$-piperideine (8.2 g), o-fluorobenzhydrazide (4.43 g), 2-methoxyethanol (96 ml) and tri-n-propylamine (3.9 ml) is stirred at reflux under nitrogen for 42 hours. It is cooled and the precipitated solid collected, washed with ethanol, dried and recrystallized from 2-methoxyethanol to afford pure 9-benzyl-2-(2-fluorophenyl)-7,8,9,10-tetrahydropyrido[3,4-e][1,2,4]triazolo[1,5-c]pyrimidin-5(6H)one, melting in the range 256 to 259°. When treated with an equimolar quantity of methanesulphonic acid in methanol, the free base is convened to the methanesulphonate salt (38 %) m.p. 306-309° after recrystallization from 1:1 dimethylacetamide-methanol mixture.

The above ethyl carbamate derivative is prepared by the method described in Example 2 from N-benzyl-3-cyano-4-amino $\Delta^3$-piperideine (Taylor et al, Tetrahedron 23, 855-890 (1967) and is obtained in 94 % yield.

When the ethyl carbamate of N-benzyl-3-cyano-4-amino-$\Delta^3$-piperidine of Example 8 of EP 0 217 748 is replaced by the ethyl carbamate of (+) or (-) N-$\alpha$-methylbenzyl-3-cyano-4-amino-$\Delta^3$-piperideine, the corresponding (+) or (-)-2-(2-fluoro)-9-$\alpha$-methylbenzyl-7,8,9,10-tetrahydropyrido[3,4-e][1,2,4]triazolo-[1,5-c]-pyrimidin-5(6H)one is obtained.

## Claims

### Claims for the following Contracting States : AT, BE, CH, DE, FR, GB, IT, LI, LU, NL, SE

**1.** A 2-substituted-e-fused [1,2,4]triazolo[1,5-c]pyrimidine compound of the formula

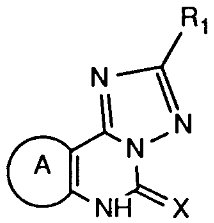

(I),

wherein

a) X is oxygen, $R_1$ is 2-fluorophenyl and ring A is $A^3$-piperideine substituted at nitrogen by methoxycarbonylmethyl, 3-picolyl, 2-picolyl, 4-picolyl, phenylsulfonyl, carboxymethyl, carbamoylmethyl, carbamoyl, cyanomethyl, tetrazolylmethyl, (+)-α-methylbenzyl or(-)-α-methylbenzyl; or

b) X is oxygen, $R_1$ is 2-chlorophenyl, 2-pyridyl or 2-pyrrolyl and ring A is N-benzyl-$\Delta^3$-piperideine ; or

c) X is imino, $R_1$ is 2-furyl and ring A is cyclopentene;

a tautomer thereof or a pharmaceutically acceptable salt of said compound or tautomer.

**2.** The compound according to claim 1 of the formula I, wherein X is oxygen, $R_1$ is 2-chlorophenyl and ring A is N-benzyl-$\Delta^3$-piperideine having the name 9-benzyl-2-(2-chlorophenyl)-7,8,9,10-tetrahydropyrido[3,4-e][1,2,4]triazolo[1,5-c]pyrimidin-5(6H)one; a tautomer thereof or a pharmaceutically acceptable salt thereof.

**3.** The compound according to claim 1 of the formula I, wherein X is oxygen, $R_1$ is 2-pyridyl and ring A is N-benzyl-$\Delta^3$-piperideine having the name 9-benzyl-2-(2-pyridyl)-7,8,9,10-tetrahydropyrido[3,4-e][1,2,4]-triazolo[1,5-c]-pyrimidin-5(6H)one; a tautomer thereof or a pharmaceutically acceptable salt thereof.

**4.** The compound according to claim 1 of the formula I, wherein X is oxygen, $R_1$ is 2-pyrrolyl and ring A is N-benzyl-$\Delta^3$-piperideine having the name 9-benzyl-2-(2-pyrrolyl)-7,8,9,10-tetrahydropyrido[3,4-e][1,2,4]-triazolo[1,5-c]pyrimidin-5(6H)one; a tautomer thereof or a pharmaceutically acceptable salt thereof.

**5.** The compound according to claim 1 of the formula I, wherein X is imino, $R_1$ is 2-furyl and ring A is N-benzyl-$\Delta^3$-piperideine having the name 2-(2-furyl)-5-imino-5,6,8,9-tetrahydro-7H-cyclopenta[e][1,2,4]-triazolo[1,5-c]-pyrimidine; a tautomer thereof or a pharmaceutically acceptable salt thereof.

**6.** The compound according to claim 1 of the formula I, wherein X is oxygen, $R_1$ is 2-fluorophenyl and ring A is N-(methoxycarbonylmethyl)-$\Delta^3$-piperideine having the name 9-carbomethoxy-methyl-2-(2-fluorophenyl)7,8,9,10-tetrahydropyrido[3,4-e][1,2,4]triazolo[1,5-c]pyrimidin-5(6H)one; a tautomer thereof or a pharmaceutically acceptable salt thereof.

**7.** The compound according to claim 1 of the formula I, wherein X is oxygen, $R_1$ is 2-fluorophenyl and ring A is N-(3-picolyl)-$\Delta^3$-piperideine having the name 2-(2-fluorophenyl)-9-(3-picolyl)-7,8,9,10-tetrahydro-pyrido[3,4-e][1,2,4]triazolo[1,5-c]pyrimidin-5(6H)one; a tautomer thereof or a pharmaceutically acceptable salt thereof.

**8.** The compound according to claim 1 of the formula I, wherein X is oxygen, $R_1$ is 2-fluorophenyl and ring A is N-(2- or 4-picolyl)-$\Delta^3$-piperideine having the name 2-(2-fluorophenyl)-9-(2-picolyl)-7,8,9,10-tetrahydro-pyrido[3,4-e][1,2,4]-triazolo[1,5-c]pyrimidin-5(6H)one or 2-(2-fluorophenyl)-9-(4-picolyl)-7,8,9,10-tetrahydropyrido[3,4-e][1,2,4]triazolo[1,5-c]pyrimidin-5(6H)one; a tautomer thereof or a pharmaceutically acceptable salt thereof.

**9.** The compound according to claim 1 of the formula I, wherein X is oxygen, $R_1$ is 2-fluorophenyl and ring A is N-phenylsulfonyl-$\Delta^3$-piperideine having the name 2-(2-fluorophenyl)-9-phenylsulphonyl-7,8,9,10-tetrahydropyrido[3,4-e][1,2,4]triazolo[1,5-c]pyrimidin-5(6H)one; a tautomer thereof or a pharmaceutically acceptable salt thereof.

**10.** The compound according to claim 1 of the formula I, wherein X is oxygen, $R_1$ is 2-fluorophenyl and

ring A is N-carboxymethyl-$\Delta^3$-piperideine having the name 9-carboxymethyl-2-(2-fluorophenyl)-7,8,9,10-tetrahydropyrido[3,4-e][1,2,4]triazolo[1,5-c] pyrimidin-5(6H)one; a tautomer thereof or a pharmaceutically acceptable salt thereof.

**11.** The compound according to claim 1 of the formula I, wherein X is oxygen, $R_1$ is 2-fluorophenyl and ring A is N-(carbamoylmethyl)-$\Delta^3$-piperideine having the name 9-carbamoylmethyl-2-(2-fluorophenyl)-7,8,9,10-tetrahydropyrido[3,4-e][1,2,4]triazolo[1,5-c]pyrimidin-5(6H)one; a tautomer thereof or a pharmaceutically acceptable salt thereof.

**12.** The compound according to claim 1 of the formula I, wherein X is oxygen, $R_1$ is 2-fluorophenyl and ring A is N-(carbamoyl)-$\Delta^3$-piperideine having the name 9-carbamoyl-2(2-fluorophenyl)-7,8,9,10-tetrahydropyrido[3,4-e][1,2,4]triazolo[1,5-c]pyrimidine-5(6H)one; a tautomer thereof or a pharmaceutically acceptable salt thereof.

**13.** The compound according to claim 1 of the formula I, wherein X is oxygen, $R_1$ is 2-fluorophenyl and ring A is N-cyanomethyl-$\Delta^3$-piperideine having the name 9-cyanomethyl-2-(2-fluorophenyl)-7,8,9,10-tetrahydropyrido[3,4-e]-[1,2,4]triazolo[1,5-c]pyrimidin-5(6H)one; a tautomer thereof or a pharmaceutically acceptable salt thereof.

**14.** The compound according to claim 1 of the formula I, wherein X is oxygen, $R_1$ is 2-fluorophenyl and ring A is N-(tetrazolylmethyl)-$\Delta^3$-piperideine having the name 2-(2-fluorophenyl)-9-tetrazolylmethyl-7,8,9,10-tetrahydropyrido[3,4-e][1,2,4]triazolo[1,5-c]pyrimidine-5(6H)one; a tautomer thereof or a pharmaceutically acceptable salt thereof.

**15.** The compound according to claim 1 of the formula I, wherein X is oxygen, $R_1$ is 2-fluorophenyl and ring A is N-((+)- or (-)-$\alpha$-methylbenzyl)-$\Delta^3$-piperideine having the name (+) or (-)-2-(2-fluoro)-9-$\alpha$-methylbenzyl-7,8,9,10-tetrahydropyrido[3,4-e][1,2,4]triazolo-[1,5-c]pyrimidin-5(6H)one; a tautomer thereof or a pharmaceutically acceptable salt thereof.

**16.** The compounds according to claims 1-15 for use in the treatment of anxiety or asthma or as adenosine antagonists.

**17.** Pharmaceutical compositions containing an effective amount of a compound according to claims 1-15 and a pharmaceutically acceptable carrier.

**18.** Process for the preparation of a compound of formula I, wherein X, $R_1$ and A are as defined in claim 1, which comprises

a) cyclizing a compound of the formula:

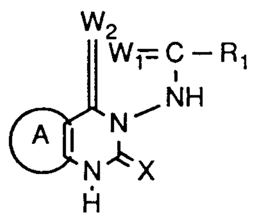

(II),

wherein $R_1$, X and ring A are as defined above, one of $W_1$ and $W_2$ is NH and the other one of $W_1$ and $W_2$ represents O or NH, by treatment with a base furnishing a nitrogen atom, or

b) to obtain a compound of formula I, wherein X is oxygen, cyclizing a compound of the formula:

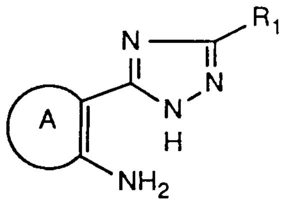

(III)

wherein $R_1$ and ring A are as defined above, by treatment with a reactive derivative of carbonic acid, or

c) to obtain a compound of formula I, wherein X is oxygen, reacting a compound of the formula:

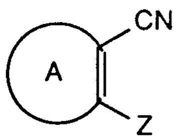

(IV)

wherein ring A is as defined above and Z is the radical of a carbonic acid derivative bonded via a nitrogen atom, with a hydrazide of the formula:

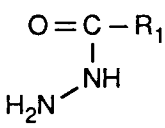

(V)

d) to obtain a compound of formula I wherein X is oxygen, treating a compound of the formula:

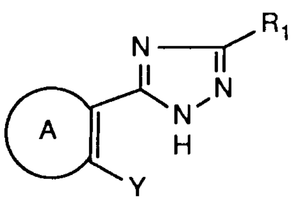

(VI)

wherein Y represents a group capable of being converted to the grouping -N=C=O by an oxidizing agent, with said oxidizing agent followed by ring closure, or

e) to obtain the compound of formula I, wherein X is NH, converting a compound of the formula:

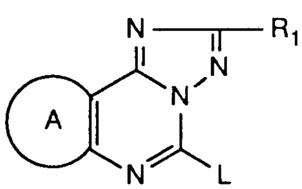

(VII)

wherein $R_1$ is 2-furyl, ring A is cyclopentane and L is selected from halogen, $C_1$-$C_7$-alkoxy, aryl-$C_1$-$C_7$-alkoxy, mercapto, $C_1$-$C_7$-alkylthio and isothiocyanato, by replacing the group L by the grouping NH, or

f) treating a compound of the formula:

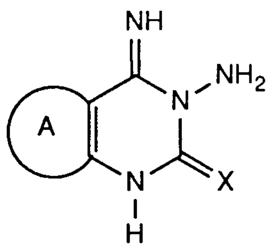

(VIII)

with a reactive derivative of a carboxylic acid of the formula $R_1$-COOH, or

g) to obtain the compound of formula I wherein X is imino, treating a compound of formula I wherein X is NR, wherein R is $C_1$-$C_7$-alkyl, $C_2$-$C_7$-alkenyl or $C_2$-$C_7$-alkynyl, with aqueous acid, or

h) to obtain the compound of formula I wherein X is NH, i) reacting a compound of formula III, as defined in process (b) above, with cyanogen halide followed by cyclization or reacting a compound of formula III with cyanamide or a reactive imino carbonyl derivative, or

i) to obtain a compound of formula I wherein X is oxygen, reacting a compound of the formula

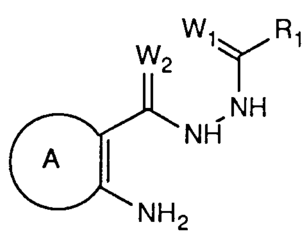

(IX)

with a reactive derivative of carbonic acid and base, or

j) to obtain a compound of formula I wherein X is oxygen, hydrolyzing a compound of the formula

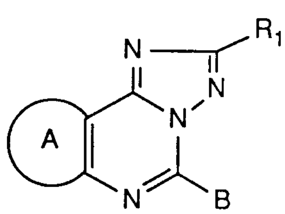

XV

wherein B is halo or $C_1$-$C_7$-alkoxy, or

k) to obtain the compound of formula I wherein X is imino, by doubly cyclizing a compound of the formula

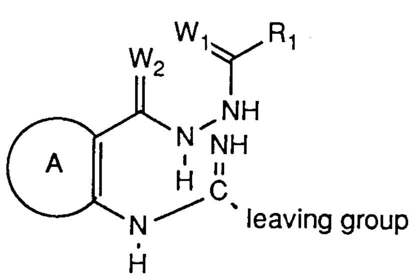

(XVI)

or the formula

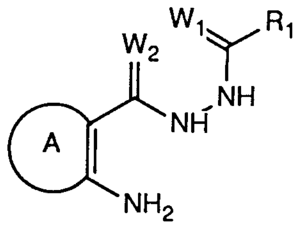

(XVII)

or the formula

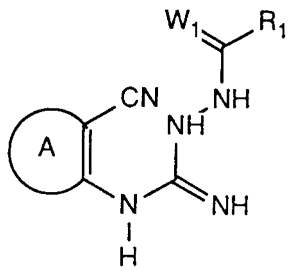

(XVIII),

the reaction of the compound of formula XVII being in the presence of a cyanogen halide or cyanamide or a reactive imino carbonic acid derivative or
l) to obtain the compound of formula I wherein X is imino, cyclizing a compound of the formula

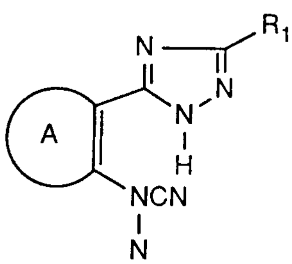

XX

which is prepared from a compound of formula III by reaction thereof with cyanogen halide, or
m) to obtain the compound of formula I wherein X is imino, reacting a compound of the formula

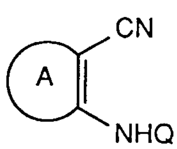

XXI

wherein Q is -CN or

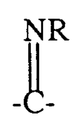

leaving group with a hydrazide of formula V;
and if desired, converting a resulting compound into another compound of the invention and/or

converting a resulting salt into the free compound or into a different salt and/or converting a resulting free compound having a salt forming group into a salt.

**Claims for the following Contracting States : ES, GR**

1. Process for the preparation of a compound of the formula

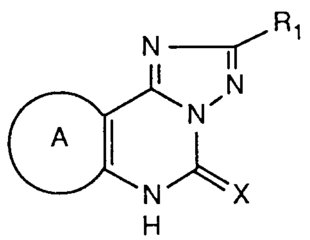

(I),

wherein

a) X is oxygen, $R_1$ is 2-fluorophenyl and ring A is $\Delta^3$-piperideine substituted at nitrogen by methoxycarbonylmethyl, 3-picolyl, 2-picolyl, 4-picolyl, phenylsulfonyl, carboxymethyl, carbamoylmethyl, carbamoyl, cyanomethyl, tetrazolylmethyl, (+)-α-methylbenzyl or (-)-α-methylbenzyl; or

b) X is oxygen, $R_1$ is 2-chlorophenyl, 2-pyridyl or 2-pyrrolyl and ring A is N-benzyl-$\Delta^3$-piperideine ; or

c) X is imino, $R_1$ is 2-furyl and ring A is cyclopentene;

a tautomer thereof or a pharmaceutically acceptable salt of said compound or tautomer, which comprises

a) cyclizing a compound of the formula:

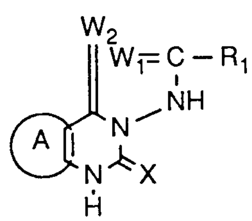

(II),

wherein $R_1$, X and ring A are as defined above, one of $W_1$ and $W_2$ is NH and the other one of $W_1$ and $W_2$ represents O or NH, by treatment with a base furnishing a nitrogen atom, or

b) to obtain a compound of formula I, wherein X is oxygen, cyclizing a compound of the formula:

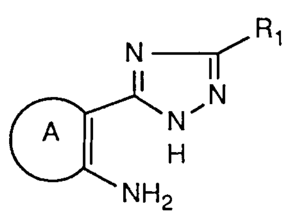

(III)

wherein $R_1$ and ring A are as defined above, by treatment with a reactive derivative of carbonic acid, or

c) to obtain a compound of formula I, wherein X is oxygen, reacting a compound of the formula:

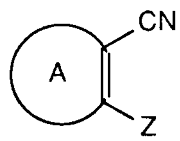

(IV)

wherein ring A is as defined above and Z is the radical of a carbonic acid derivative bonded via a nitrogen atom, with a hydrazide of the formula:

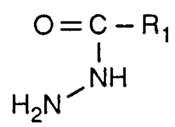

(V)

d) to obtain a compound of formula I wherein X is oxygen, treating a compound of the formula:

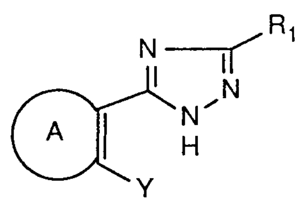

(VI)

wherein Y represents a group capable of being converted to the grouping -N = C = O by an oxidizing agent, with said oxidizing agent followed by ring closure, or

e) to obtain the compound of formula I, wherein X is NH, converting a compound of the formula:

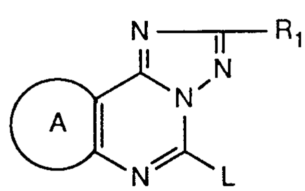

(VII)

wherein $R_1$ is 2-furyl, ring A is cyclopentane and L is selected from halogen, $C_1$-$C_7$-alkoxy, aryl-$C_1$-$C_7$-alkoxy, mercapto, $C_1$-$C_7$-alkylthio and isothiocyanato, by replacing the group L by the grouping NH, or

f) treating a compound of the formula:

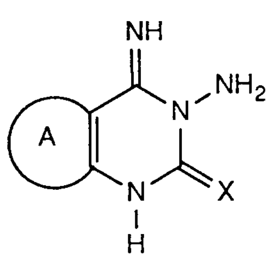

(VIII)

with a reactive derivative of a carboxylic acid of the formula $R_1$-COOH,

g) to obtain the compound of formula I wherein X is imino, treating a compound of formula I wherein X is NR, wherein R is $C_1$-$C_7$-alkyl, $C_2$-$C_7$-alkenyl or $C_2$-$C_7$-alkynyl, with aqueous acid, or

h) to obtain the compound of formula I wherein X is NH, i) reacting a compound of formula III, as defined in process (b) above, with cyanogen halide followed by cyclization or reacting a compound of formula III with cyanamide or a reactive imino carbonyl derivative, or

i) to obtain a compound of formula I wherein X is oxygen, reacting a compound of the formula

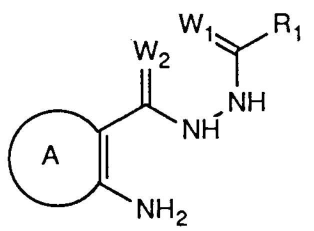

(IX)

with a reactive derivative of carbonic acid and base, or

j) to obtain a compound of formula I wherein X is oxygen, hydrolyzing a compound of the formula

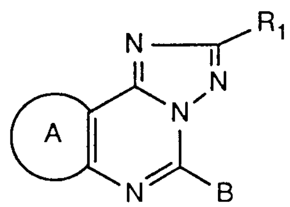

XV

wherein B is halo or $C_1$-$C_7$-alkoxy, or

k) to obtain the compound of formula I wherein X is imino, by doubly cyclizing a compound of the formula

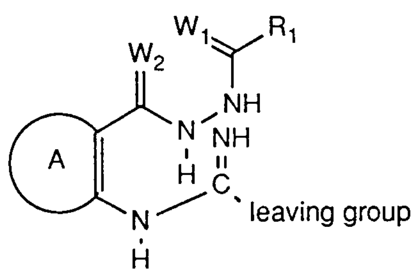

(XVI)

or the formula

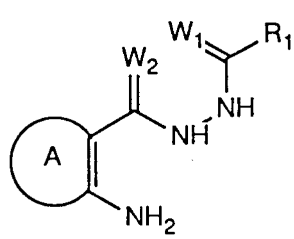

(XVII)

or the formula

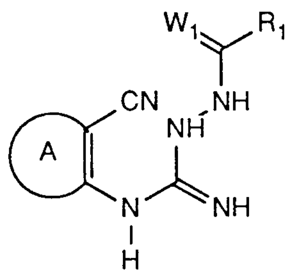

(XVIII),

the reaction of the compound of formula XVII being in the presence of a cyanogen halide or cyanamide or a reactive imino carbonic acid derivative or
l) to obtain the compound of formula I wherein X is imino, cyclizing a compound of the formula

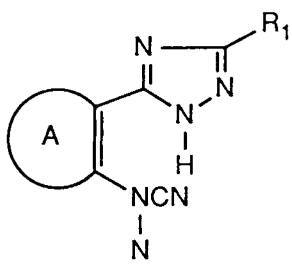

XX

which is prepared from a compound of formula III by reaction thereof with cyanogen halide, or
m) to obtain the compound of formula I where in X is imino, reacting a compound of the formula

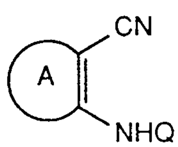

XXI

wherein Q is -CN or

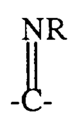

leaving group with a hydrazide of formula V;
and if desired, converting a resulting compound into another compound of the invention and/or converting a resulting salt into the free compound or into a different salt and/or converting a resulting free compound having a salt forming group into a salt.

**2.** Process according to claim I, wherein the starting materials are chosen so that the compound of the formula I, wherein X is oxygen, $R_1$ is 2-chlorophenyl and ring A is N-benzyl-$\Delta^3$-piperideine having the name 9-benzyl-2-(2-chlorophenyl)-7,8,9,10-tetrahydropyrido[3,4-e][1,2,4]triazolo[1,5-c]pyrimidin-5(6H)-one; a tautomer thereof or a pharmaceutically acceptable salt thereof is manufactured.

**3.** Process according to claim I, wherein the starting materials are chosen so that the compound of the formula I, wherein X is oxygen, $R_1$ is 2-pyridyl and ring A is N-benzyl-$\Delta^3$-piperideine having the name 9-benzyl-2-(2-pyridyl)-7,8,9,10-tetrahydropyrido[3,4-e][1,2,4]triazolo[1,5-c]-pyrimidin-5(6H)one; a tautomer thereof or a pharmaceutically acceptable salt thereof is manufactured.

**4.** Process according to claim I, wherein the starting materials are chosen so that the compound of the formula I, wherein X is oxygen, $R_1$ is 2-pyrrolyl and ring A is N-benzyl-$\Delta^3$-piperideine having the name 9-benzyl-2-(2-pyrrolyl)-7,8,9,10-tetrahydropyrido[3,4-e][1,2,4]triazolo[1,5-c]pyrimidin-5(6H)one; a tautomer thereof or a pharmaceutically acceptable salt thereof is manufactured.

**5.** Process according to claim I, wherein the starting materials are chosen so that the compound of the formula I, wherein X is imino, $R_1$ is 2-furyl and ring A is N-benzyl-$\Delta^3$-piperideine having the name 2-(2-furyl)-5-imino-5,6,8,9-tetrahydro-7H-cyclopenta[e][1,2,4]triazolo[1,5-c]-pyrimidine; a tautomer thereof or a pharmaceutically acceptable salt thereof is manufactured.

**6.** Process according to claim I, wherein the starting materials are chosen so that the compound of the formula I, wherein X is oxygen, $R_1$ is 2-fluorophenyl and ring A is N-(methoxycarbonylmethyl)-$\Delta^3$-piperideine having the name 9-carbomethoxy-methyl-2-(2-fluorophenyl)-7,8,9,10-tetrahydropyrido[3,4-e]-[1,2,4]triazolo[1,5-c]pyrimidin-5(6H)one; a tautomer thereof or a pharmaceutically acceptable salt thereof is manufactured.

**7.** Process according to claim I, wherein the starting materials are chosen so that the compound of the formula I, wherein X is oxygen, $R_1$ is 2-fluorophenyl and ring A is N-(3-picolyl)$\Delta^3$-piperideine having the name 2-(2-fluorophenyl)-9-(3-picolyl)-7,8,9,10-tetrahydro-pyrido[3,4-e][1,2,4]triazolo[1,5-c]pyrimidin-5-(6H)one; a tautomer thereof or a pharmaceutically acceptable salt thereof is manufactured.

**8.** Process according to claim I, wherein the starting materials are chosen so that the compound of the formula I, wherein X is oxygen, $R_1$ is 2-fluorophenyl and ring A is N-(2- or 4-picolyl)$\Delta^3$-piperideine having the name 2(2-fluorophenyl)-9-(2-picolyl)-7,8,9,10-tetrahydropyrido[3,4-e][1,2,4]triazolo[1,5-c]-pyrimidin-5(6H)one or 2(2-fluorophenyl)-9-(4picolyl)-7,8,9,10-tetrahydropyrido[3,4-e][1,2,4]triazolo[1,5-c]-pyrimidin-5(6H)one; a tautomer thereof or a pharmaceutically acceptable salt thereof is manufactured.

**9.** Process according to claim I, wherein the starting materials are chosen so that the compound of the formula I, wherein X is oxygen, $R_1$ is 2-fluorophenyl and ring A is N-phenylsulfonyl-$\Delta^3$-piperideine having the name 2-(2-fluorophenyl)-9-phenylsulphonyl-7,8,9,10-tetrahydropyrido[3,4-e][1,2,4]triazolo[1,5-c]pyrimidin-5(6H)one; a tautomer thereof or a pharmaceutically acceptable salt thereof is manufactured.

**10.** Process according to claim I, wherein the starting materials are chosen so that the compound of the formula I, wherein X is oxygen, $R_1$ is 2-fluorophenyl and ring A is N-carboxymethyl-$\Delta^3$piperideine having the name 9-carboxymethyl-2-(2-fluorophenyl)-7,8,9,10-tetrahydropyrido[3,4-e][1,2,4]triazolo[1,5-c]pyrimidin-5(6H)one; a tautomer thereof or a pharmaceutically acceptable salt thereof is manufactured.

**11.** Process according to claim I, wherein the starting materials are chosen so that the compound of the formula I, wherein X is oxygen, $R_1$ is 2-fluorophenyl and ring A is N-(carbamoylmethyl)$A^3$-piperideine having the name 9-carbamoylmethyl-2-(2-fluorophenyl)-7,8,9,10-tetrahydropyrido[3,4-e][1,2,4]triazolo-[1,5-c]pyrimidin-5(6H)one; a tautomer thereof or a pharmaceutically acceptable salt thereof is manufactured.

**12.** Process according to claim I, wherein the starting materials are chosen so that the compound of the formula I, wherein X is oxygen, $R_1$ is 2-fluorophenyl and ring A is N-(carbamoyl)$\Delta^3$-piperideine having the name 9-carbamoyl-2-(2-fluorophenyl)-7,8,9,10-tetrahydropyrido[3,4-e][1,2,4]triazolo[1,5-c]pyrimidine-5(6H)one; a tautomer thereof or a pharmaceutically acceptable salt thereof is manufactured.

**13.** Process according to claim I, wherein the starting materials are chosen so that the compound of the formula I, wherein X is oxygen, $R_1$ is 2-fluorophenyl and ring A is N-cyanomethyl$\Delta^3$-piperideine having the name 9-cyanomethyl-2-(2-fluorophenyl)-7,8,9,10-tetrahydropyrido[3,4-e]-[1,2,4]triazolo[1,5-c]-pyrimidin-5(6H)one; a tautomer thereof or a pharmaceutically acceptable salt thereof is manufactured.

**14.** Process according to claim I, wherein the starting materials are chosen so that the compound of the formula I, wherein X is oxygen, $R_1$ is 2-fluorophenyl and ring A is N-(tetrazolylmethyl)-$\Delta^3$-piperideine having the name 2-(2-fluorophenyl)-9-tetrazolylmethyl-7,8,9,10tetrahydropyrido[3,4-e][1,2,4]triazolo [1,5-c]pyrimidine-5(6H)one; a tautomer thereof or a pharmaceutically acceptable salt thereof is manufactured.

15. Process according to claim I, wherein the starting materials are chosen so that the compound of the formula I, wherein X is oxygen, $R_1$ is 2-fluorophenyl and ring A is N-((+)- or (-)-αmethylbenzyl)-$\Delta^3$-piperideine having the name (+) or (-)-2-(2-fluoro)-9-α-methylbenzyl7,8,9,10-tetrahydropyrido[3,4-e]-[1,2,4]triazolo-[1,5-c]pyrimidin-5(6H)one; a tautomer thereof or a pharmaceutically acceptable salt thereof is manufactured.

**Revendications**

**Revendications pour les Etats contractants suivants : AT, BE, CH, DE, FR, GB, IT, LI, LU, NL, SE**

1. Composé [1,2,4]triazolo[1,5-c]pyrimidine e-condensé 2-substitué de formule

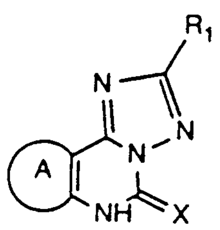

(I),

dans laquelle

a) X est un oxygène, $R_1$ est un groupe 2-fluorophényle et le noyau A est une $\Delta^3$-pipéridéine substituée sur l'atome d'azote par un groupe méthoxycarbonylméthyle, 3-picolyle, 2-picolyle, 4-picolyle, phénylsulfonyle, carboxyméthyle, carbamoylméthyle, carbamoyle, cyanométhyle, tétrazolylméthyle, (+)-α-méthylbenzyle ou (-)-α-méthylbenzyle; ou

b) X est un oxygène, $R_1$ est un groupe 2-chlorophényle, 2-pyridyle ou 2-pyrrolyle et le noyau A est un groupe N-benzoyl-$\Delta^3$-pipéridéine; ou

c) X est un groupe imino, $R_1$ est un groupe 2-furyle et le noyau A est un cyclopentène; un de ses tautomères ou un sel pharmaceutiquement acceptable dudit composé ou tautomère.

2. Composé de formule I selon la revendication 1, dans lequel X est un oxygène, $R_1$ est un groupe 2-chlorophényle et le noyau A est la N-benzyl-$\Delta^3$-pipéridéine, dont le nom est la 9-benzyl-2-(2-chlorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

3. Composé de formule I selon la revendication 1, dans lequel X est un oxygène, $R_1$ est un groupe 2-pyridyle et le noyau A est la N-benzyl-$\Delta^3$-pipéridéine, dont le nom est la 9-benzyl-2-(2-pyridyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

4. Composé de formule I selon la revendication 1, dans lequel X est un oxygène, $R_1$ est un groupe 2-pyrrolyle et le noyau A est la N-benzyl-$\Delta^3$-pipéridéine, dont le nom est la 9-benzyl-2-(2-pyrrolyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

5. Composé de formule I selon la revendication 1, dans lequel X est un groupe imino, $R_1$ est un groupe 2-furyle et le noyau A est la N-benzyl-$\Delta^3$-pipéridéine, dont le nom est la 2-(2-furyl)-5-imino-5,6,8,9-tétrahydro-7H-cyclopenta[e][1,2,4]triazolo[1,5-c]pyrimidine; un de ses tautomères ou un de ses sels pharmaceutiquement acceptables.

6. Composé de formule I selon la revendication 1, dans lequel X est un oxygène, $R_1$ est un groupe 2-fluorophényle et le noyau A est la N-(méthoxycarbonylméthyl)-$\Delta^3$-pipéridéine, dont le nom est la 9-carbométhoxyméthyl-2-(2-fluorophényl)-7,8,9,10-tétrahydropyrido[3,4-e][1,2,4]triazolo[1,5-c]pyrimidine-5(6H)one;un de ses tautomères ou un de ses sels pharmaceutiquement acceptables.

7. Composé de formule I selon la revendication 1, dans lequel X est un oxygène, $R_1$ est un groupe 2-fluorophényle et le noyau A est la N-(3-picolyl)-$\Delta^3$-pipéridéine, dont le nom est la 2-(2-fluorophényl)-9-(3-picolyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**8.** Composé de formule I selon la revendication 1, dans lequel X est un oxygène, $R_1$ est un groupe 2-fluorophényle et le noyau A est la N-(2- ou 4-picolyl)-$\Delta^3$-pipéridéine, dont le nom est la 2-(2-fluorophényl)-9-(2-picolyl)-7,8,9,10-tétrahydropyrido[3,4-e][1,2,4]triazolo[1,5-c]pyrimidine-5(6H)one ou la 2-(2-fluorophényl)-9-(4-picolyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**9.** Composé de formule I selon la revendication 1, dans lequel X est un oxygène, $R_1$ est un groupe 2-fluorophényle et le noyau A est la N-phénylsulfonyl-$\Delta^3$-pipéridéine, dont le nom est la 2-(2-fluorophényl)-9-phénylsulfonyl-7,8,9,10-tétrahydropyrido[3,4-e][1,2,4]triazolo[1,5-c]pyrimidine-5(6H)one; un de ses tautomères ou un de ses sels pharmaceutiquement acceptables.

**10.** Composé de formule I selon la revendication 1, dans lequel X est un oxygène, $R_1$ est un groupe 2-fluorophényle et le noyau A est la N-carboxyméthyl-$\Delta^3$-pipéridéine, dont le nom est la 9-carboxyméthyl-2-(2-fluorophényl)-7,8,9,10-tétrahydropyrido[3,4-e][1,2,4]triazolo[1,5-c]pyrimidine-5(6H)-one; un de ses tautomères ou un de ses sels pharmaceutiquement acceptables.

**11.** Composé de formule I selon la revendication 1, dans lequel X est un oxygène, $R_1$ est un groupe 2-fluorophényle et le noyau A est la N-(carbamoylméthyl)-$\Delta^3$-pipéridéine, dont le nom est la 9-carbamoylméthyl-2-(2-fluorophényl)-7,8,9,10-tétrahydropyrido[3,4-e][1,2,4]triazolo[1,5-c]pyrimidine-5-(6H)one; un de ses tautomères ou un de ses sels pharmaceutiquement acceptables.

**12.** Composé de formule I selon la revendication 1, dans lequel X est un oxygène, $R_1$ est un groupe 2-fluorophényle et le noyau A est la N-carbamoyl-$\Delta^3$-pipéridéine, dont le nom est la 9-carbamoyl-2-(2-fluorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**13.** Composé de formule I selon la revendication 1, dans lequel X est un oxygène, $R_1$ est un groupe 2-fluorophényle et le noyau A est la N-cyanométhyl-$\Delta^3$-pipéridéine, dont le nom est la 9-cyanométhyl-2-(2-fluorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**14.** Composé de formule I selon la revendication 1, dans lequel X est un oxygène, $R_1$ est un groupe 2-fluorophényle et le noyau A est la N-(tétrazolylméthyl)-$\Delta^3$-pipéridéine, dont le nom est la 2-(2-fluorophényl)-9-tétrazolylméthyl-7,8,9,10-tétrahydropyrido[3,4-e][1,2,4]triazolo[1,5-c]pyrimidine-5(6H)one; un de ses tautomères ou un de ses sels pharmaceutiquement acceptables.

**15.** Composé de formule I selon la revendication 1, dans lequel X est un oxygène, $R_1$ est un groupe 2-fluorophényle et le noyau A est la N-((+)- ou (-)-$\alpha$-méthylbenzyl)-$\Delta^3$-pipéridéine, dont le nom est la (+)- ou (-)-2-(2-fluorophényl)-9-$\alpha$-méthylbenzyl-7,8,9,10-tétrahydropyrido[3,4-e][1,2,4]triazolo[1,5-c]-pyrimidine-5(6H)one; un de ses tautomères ou un de ses sels pharmaceutiquement acceptables.

**16.** Composés selon les revendications 1-15, utilisables dans le traitement de l'anxiété ou de l'asthme ou comme antagonistes d'adénosine.

**17.** Compositions pharmaceutiques contenant une quantité efficace d'un composé selon les revendications 1-15 et un véhicule pharmaceutiquement acceptable.

**18.** Procédé de préparation d'un composé de formule I, dans lequel X, $R_1$ et A sont tels que définis dans la revendication 1, qui comprend

a) la cyclisation d'un composé de formule :

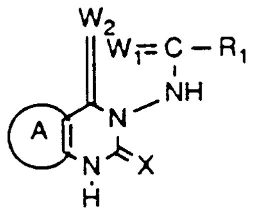

(II),

dans laquelle $R_1$, X et le noyau A sont tels que définis cidessus, l'un des radicaux $W_1$ et $W_2$ est NH et l'autre des radicaux $W_1$ et $W_2$ représente O ou NH, par traitement avec une base fournissant un atome d'azote, ou

b) pour obtenir un composé de formule I dans lequel X est un oxygène, la cyclisation d'un composé de formule

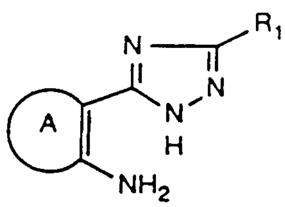

(III)

dans laquelle $R_1$ et le noyau A sont tels que définis ci-dessus, avec un dérivé réactif d'acide carbonique, ou

c) pour obtenir un composé de formule I dans lequel X est un oxygène, la réaction d'un composé de formule :

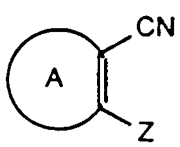

(IV)

dans laquelle le noyau A est tel que défini ci-dessus et Z est le radical d'un dérivé d'acide carbonique lié par un atome d'azote, avec un hydrazide de formule :

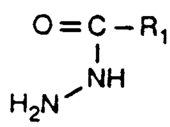

(V)

d) pour obtenir un composé de formule I dans lequel X est un oxygène, le traitement d'un composé de formule :

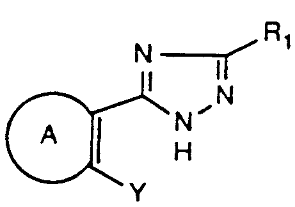

(VI)

dans laquelle Y représente un groupe capable d'être transformé en groupement -N=C=O par un agent oxydant, avec ledit agent oxydant, suivi d'une cyclisation, ou

e) pour obtenir le composé de formule I dans lequel X est NH, la transformation d'un composé de formule :

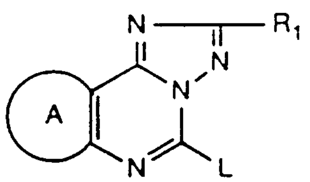

(VII)

dans laquelle $R_1$ est un groupe 2-furyle, le noyau A est le cyclopentane et L est choisi parmi un halogène, un groupe alcoxy en $C_1$-$C_7$, aryl(alcoxy en $C_1$-$C_7$), mercapto, alkylthio en $C_1$-$C_7$ et isothiocyanato, en remplaçant le groupe L par le groupement NH; ou

f) le traitement d'un composé de formule :

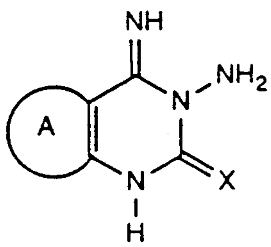

(VIII)

avec un dérivé réactif d'un acide carboxylique de formule $R_1$-COOH, ou

g) pour obtenir le composé de formule I dans lequel X est un groupe imino, le traitement d'un composé de formule I dans lequel X est NR, où R est un groupe alkyle en $C_1$-$C_7$,alcényle en $C_2$-$C_7$ ou alcynyle en $C_2$-$C_7$, avec un acide aqueux; ou

h) pour obtenir le composé de formule I dans lequel X est NH, i) la réaction d'un composé de formule III, telle que définie dans le procédé (b) ci-dessus, avec de l'halogénure de cyanogène, suivie d'une cyclisation ou de la réaction d'un composé de formule III avec le cyanamide ou un dérivé iminocarbonyle réactif; ou

i) pour obtenir un composé de formule I dans lequel X est un oxygène, la réaction d'un composé de formule

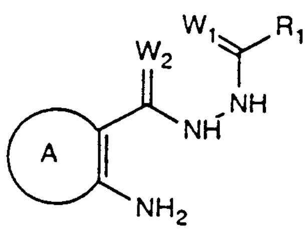

(IX)

avec un dérivé réactif d'acide carbonique et d'une base; ou

j) pour obtenir un composé de formule I dans lequel X est un oxygène, l'hydrolyse d'un composé de formule

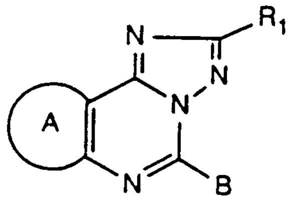

XV

dans laquelle B est un groupe halogéno ou alcoxy en $C_2$-$C_7$;ou

k) pour obtenir le composé de formule I dans lequel X est un groupe imino, la double cyclisation d'un composé de formule

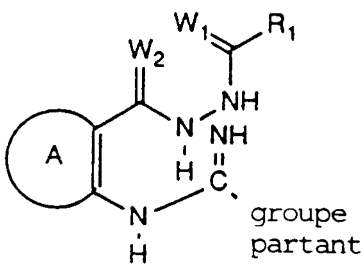

(XVI)

ou de formule

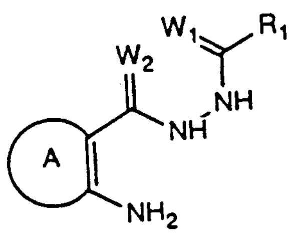

(XVII)

ou de formule

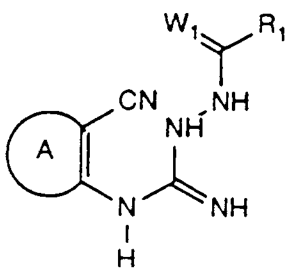

(XVIII),

la réaction du composé de formule XVII se faisant en présence d'un halogénure de cyanogène ou de cyanamide, ou d'un dérivé acide iminocarbonique réactif; ou

l) pour obtenir le composé de formule I dans lequel X est un groupe imino, la cyclisation d'un composé de formule

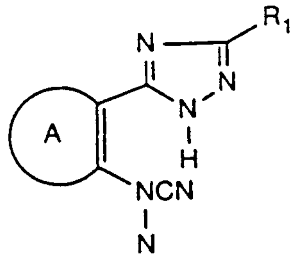

XX

que l'on prépare à partir d'un composé de formule III en le faisant réagir avec un halogénure de cyanogène; ou

m) pour obtenir le composé de formule I dans lequel X est un groupe imino, la réaction d'un composé de formule

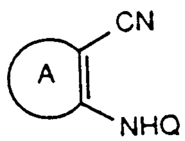

XXI

dans laquelle Q est un groupe partant -CN ou

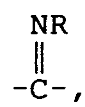

avec un hydrazide de formule V;

et, éventuellement, la transformation d'un composé obtenu en un autre composé de l'invention et/ou la transformation d'un sel obtenu en composé libre ou en un sel différent et/ou la transformation d'un composé libre obtenu possédant un groupe formant un sel en un sel.

**Revendications pour les Etats contractants suivants : ES, GR**

**1.** Procédé de préparation d'un composé de formule

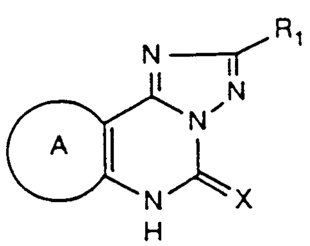

(I),

dans laquelle

a) X est un oxygène, $R_1$ est un groupe 2-fluorophényle et le noyau A est une $\Delta^3$-pipéridéine substituée sur l'atome d'azote par un groupe méthoxycarbonylméthyle, 3-picolyle, 2-picolyle, 4-picolyle, phénylsulfonyle, carboxyméthyle, carbamoylméthyle, carbamoyle, cyanométhyle, tétrazolylméthyle, (+)-α-méthylbenzyle ou (-)-α-méthylbenzyle; ou

b) X est un oxygène, $R_1$ est un groupe 2-chlorophényle, 2-pyridyle ou 2-pyrrolyle et le noyau A est un groupe N-benzoyl-$\Delta^3$-pipéridéine; ou

c) X est un groupe imino, $R_1$ est un groupe 2-furyle et le noyau A est un cyclopentène;

d'un de ses tautomères ou d'un sel pharmaceutiquement acceptable dudit composé ou tautomère, qui comprend

a) la cyclisation d'un composé de formule :

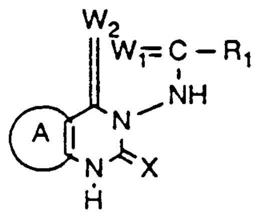

(II),

dans laquelle $R_1$, X et le noyau A sont tels que définis ci-dessus, l'un des radicaux $W_1$ et $W_2$ est NH et l'autre des radicaux $W_1$ et W2 représente O ou NH, par traitement avec une base fournissant un atome d'azote, ou

b) pour obtenir un composé de formule I dans lequel X est un oxygène, la cyclisation d'un composé de formule

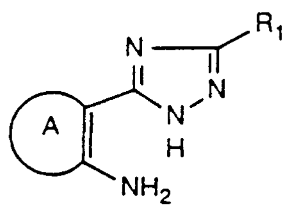

(III)

dans laquelle $R_1$ et le noyau A sont tels que définis ci-dessus, avec un dérivé réactif d'acide carbonique, ou

c) pour obtenir un composé de formule I dans lequel X est un oxygène, la réaction d'un composé de formule :

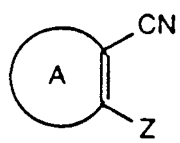

(IV)

dans laquelle le noyau A est tel que défini ci-dessus et Z est le radical d'un dérivé d'acide carbonique lié par un atome d'azote, avec un hydrazide de formule :

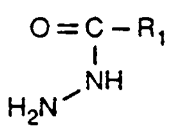

(V)

d) pour obtenir un composé de formule I dans lequel X est un oxygène, le traitement d'un composé de formule :

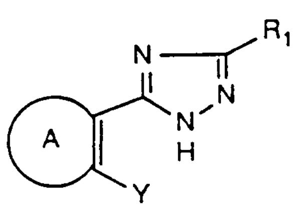

(VI)

dans laquelle Y représente un groupe capable d'être transformé en groupement -N=C=O par un agent oxydant, avec ledit agent oxydant, suivi d'une cyclisation, ou

e) pour obtenir le composé de formule I dans lequel X est NH, la transformation d'un composé de formule :

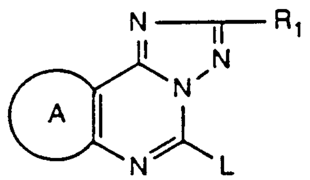

(VII)

dans laquelle $R_1$ est un groupe 2-furyle, le noyau A est le cyclopentane et L est choisi parmi un halogène, un groupe alcoxy en $C_1$-$C_7$, aryl(alcoxy en $C_1$-$C_7$), mercapto, alkylthio en $C_1$-$C_7$ et isothiocyanato, en remplaçant le groupe L par le groupement NH; ou

f) le traitement d'un composé de formule :

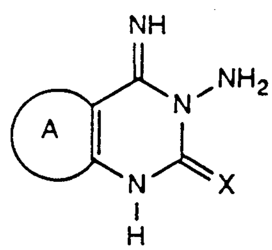

(VIII)

avec un dérivé réactif d'un acide carboxylique de formule $R_1$-COOH, ou

g) pour obtenir le composé de formule I dans lequel X est un groupe imino, le traitement d'un composé de formule I dans lequel X est NR, où R est un groupe alkyle en $C_1$-$C_7$,alcényle en $C_2$-$C_7$ ou alcynyle en $C_2$-$C_7$, avec un acide aqueux; ou

h) pour obtenir le composé de formule I dans lequel X est NH, i) la réaction d'un composé de formule III, telle que définie dans le procédé (b) ci-dessus, avec de l'halogénure de cyanogène, suivie d'une cyclisation ou de la réaction d'un composé de formule III avec le cyanamide ou un dérivé iminocarbonyle réactif; ou

i) pour obtenir un composé de formule I dans lequel X est un oxygène, la réaction d'un composé de formule

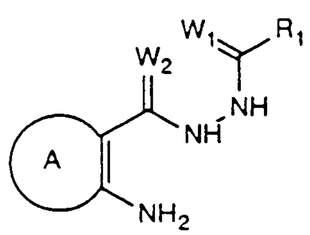

(IX)

avec un dérivé réactif d'acide carbonique et d'une base; ou

j) pour obtenir un composé de formule I dans lequel X est un oxygène, l'hydrolyse d'un composé de formule

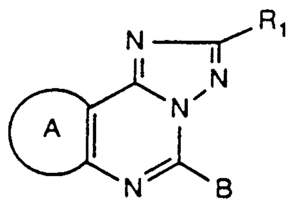

XV

dans laquelle B est un groupe halogéno ou alcoxy en $C_1$-$C_7$;ou

k) pour obtenir le composé de formule I dans lequel X est un groupe imino, la double cyclisation d'un composé de formule

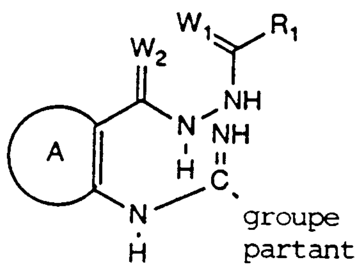

(XVI)

ou de formule

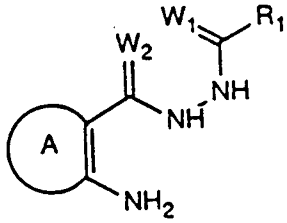

(XVII)

ou de formule

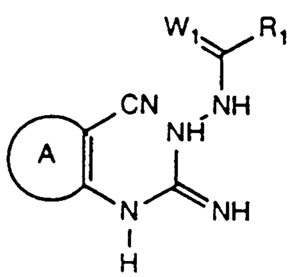

(XVIII),

la réaction du composé de formule XVII se faisant en présence d'un halogénure de cyanogène ou de cyanamide, ou d'un dérivé acide iminocarbonique réactif; ou

l) pour obtenir le composé de formule I dans lequel X est un groupe imino, la cyclisation d'un composé de formule

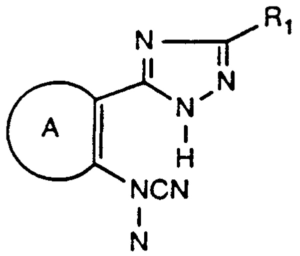

XX

que l'on prépare à partir d'un composé de formule III en le faisant réagir avec un halogénure de cyanogène; ou

m) pour obtenir le composé de formule I dans lequel X est un groupe imino, la réaction d'un composé de formule

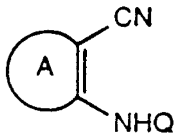

XXI

dans laquelle Q est un groupe partant -CN ou

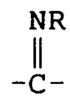

,

avec un hydrazide de formule V;

et, éventuellement, la transformation d'un composé obtenu en un autre composé de l'invention et/ou la transformation d'un sel obtenu en composé libre ou en un sel différent et/ou la transformation d'un composé libre obtenu possédant un groupe formant un sel en un sel.

**2.** Procédé selon la revendication 1, dans lequel les matières de départ sont choisies de manière à fabriquer le composé de formule I dans lequel X est un oxygène, $R_1$ est un groupe 2-chlorophényle et le noyau A est la N-benzyl-$\Delta^3$-pipéridéine, dont le nom est la 9-benzyl-2-(2-chlorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**3.** Procédé selon la revendication 1, dans lequel les matières de départ sont choisies de manière à fabriquer le composé de formule I dans lequel X est un oxygène, $R_1$ est un groupe 2-pyridyle et le noyau A est la N-benzyl-$\Delta^3$-pipéridéine, dont le nom est la 9-benzyl-2-(2-pyridyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**4.** Procédé selon la revendication 1, dans lequel les matières de départ sont choisies de manière à fabriquer le composé de formule I dans lequel X est un oxygène, $R_1$ est un groupe 2-pyrrolyle et le noyau A est la N-benzyl-$\Delta^3$-pipéri-déine, dont le nom est la 9-benzyl-2-(2-pyrrolyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

**5.** Procédé selon la revendication 1, dans lequel les matières de départ sont choisies de manière à fabriquer le composé de formule I dans lequel X est un groupe imino, $R_1$ est un groupe 2-furyle et le noyau A est la N-benzyl-$\Delta^3$-pipéridéine, dont le nom est la 2-(2-furyl)-5-imino-5,6,8,9-tétrahydro-7H-cyclopenta[e][1,2,4]triazolo[1,5-c]pyrimidine; un de ses tautomères ou un de ses sels pharmaceutiquement acceptables.

6. Procédé selon la revendication 1, dans lequel les matières de départ sont choisies de manière à fabriquer le composé de formule I dans lequel X est un oxygène, $R_1$ est un groupe 2-fluorophényle et le noyau A est la N-(méthoxycarbonylméthyl)-$\Delta^3$-pipéridéine, dont le nom est la 9-carbométhoxyméthyl-2-(2-fluorophényl)-7,8,9,10-tétrahydropyrido[3,4-e][1,2,4]triazolo[1,5-c]pyrimidine-5(6H)one;un de ses tautomères ou un de ses sels pharmaceutiquement acceptables.

7. Procédé selon la revendication 1, dans lequel les matières de départ sont choisies de manière à fabriquer le composé de formule I dans lequel X est un oxygène, $R_1$ est un groupe 2-fluorophényle et le noyau A est la N-(3-picolyl)-$\Delta^3$-pipéridéine, dont le nom est la 2-(2-fluorophényl)-9-(3-picolyl)-7,8,9,10-tétrahydropyrido[3,4-e][1,2,4]triazolo[1,5-c[pyrimidine-5(6H)one; un de ses tautomères ou un de ses sels pharmaceutiquement acceptables.

8. Procédé selon la revendication 1, dans lequel les matières de départ sont choisies de manière à fabriquer le composé de formule I dans lequel X est un oxygène, $R_1$ est un groupe 2-fluorophényle et le noyau A est la N-(2- ou 4-picolyl)-$\Delta^3$-pipéridéine, dont le nom est la 2-(2-fluorophényl)-9-(2-picolyl)-7,8,9,10-tétrahydropyrido[3,4-e][1,2,4]tri azolo[1,5-c]pyrimidine-5(6H)one ou la 2-(2-fluorophényl)-9-(4-picolyl)-7,8,9,10-tétrahydropyrido[3,4-e][1,2,4]triazolo[1,5-c]pyrimidine-5(6H)one; un de ses tautomères ou un de ses sels pharmaceutiquement acceptables.

9. Procédé selon la revendication 1, dans lequel les matières de départ sont choisies de manière à fabriquer le composé de formule I dans lequel X est un oxygène, $R_1$ est un groupe 2-fluorophényle et le noyau A est la N-phénylsulfonyl-$\Delta^3$-pipéridéine, dont le nom est la 2-(2-fluorophényl)-9-phénylsulfonyl-7,8,9,10-tétrahydropyrido[3,4-e][1,2,4]triazolo[1,5-c]pyrimidine-5(6H)one; un de ses tautomères ou un de ses sels pharmaceutiquement acceptables.

10. Procédé selon la revendication 1, dans lequel les matières de départ sont choisies de manière à fabriquer le composé de formule I dans lequel X est un oxygène, $R_1$ est un groupe 2-fluorophényle et le noyau A est la N-carboxyméthyl-$\Delta^3$-pipéridéine, dont le nom est la 9-carboxyméthyl-2-(2-fluorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

11. Procédé selon la revendication 1, dans lequel les matières de départ sont choisies de manière à fabriquer le composé de formule I dans lequel X est un oxygène, $R_1$ est un groupe 2-fluorophényle et le noyau A est la N-(carbamoylméthyl)-$\Delta^3$-pipéridéine, dont le nom est la 9-carbamoylméthyl-2-(2-fluorophényl)-7,8,9,10-tétrahydropyrido[3,4-e][1,2,4]triazolo[1,5-c]pyrimidine-5(6H)one; un de ses tautomères ou un de ses sels pharmaceutiquement acceptables.

12. Procédé selon la revendication 1, dans lequel les matières de départ sont choisies de manière à fabriquer le composé de formule I dans lequel X est un oxygène, $R_1$ est un groupe 2-fluorophényle et le noyau A est la N-carbamoyl-$\Delta^3$-pipéridéine, dont le nom est la 9-carbamoyl-2-(2-fluorophényl)-7,8,9,10-tétrahydropyrido[3,4-e][1,2,4]triazolo[1,5-c] pyrimidine-5(6H)one; un de ses tautomères ou un de ses sels pharmaceutiquement acceptables.

13. Procédé selon la revendication 1, dans lequel les matières de départ sont choisies de manière à fabriquer le composé de formule I dans lequel X est un oxygène, $R_1$ est un groupe 2-fluorophényle et le noyau A est la N-cyanométhyl-$\Delta^3$-pipéridéine, dont le nom est la 9-cyanométhyl-2-(2-fluorophényl)-7,8,9,10-tétrahydropyrido[3,4-e][1,2,4]triazolo[1,5-c[pyrimidine-5(6H)one; un de ses tautomères ou un de ses sels pharmaceutiquement acceptables.

14. Procédé selon la revendication 1, dans lequel les matières de départ sont choisies de manière à fabriquer le composé de formule I dans lequel X est un oxygène, $R_1$ est un groupe 2-fluorophényle et le noyau A est la N-(tétrazolylméthyl)-$\Delta^3$-pipéridéine, dont le nom est la 2-(2-fluorophényl)-9-tétrazolylméthyl-7,8,9,10-tétrahydropyrido[3,4-e][1,2,4]triazolo[1,5-c]pyrimidine-5(6H)one; un de ses tautomères ou un de ses sels pharmaceutiquement acceptables.

15. Procédé selon la revendication 1, dans lequel les matières de départ sont choisies de manière à fabriquer le composé de formule I dans lequel X est un oxygène, $R_1$ est un groupe 2-fluorophényle et le noyau A est la N-((+)- ou (-)-$\alpha$-méthylbenzyl)-$\Delta^3$-pipéridéine, dont le nom est la (+)- ou (-)-2-(2-fluorophényl)-9-α-méthylbenzyl-7,8,9,10-tétrahydropyrido[3,4-e][1,2,4]triazolo[1,5-c]pyrimidine-5(6H)one; un de ses tautomères ou un de ses sels pharmaceutiquement acceptables.

**Patentansprüche**

**Patentansprüch für folgende Vertragsstaaten : AT, BE, CH, DE, FR, GB, IT, LI, LU, NL, SE**

1. 2-Substituierte-e-kondensierte[1,2,4]Triazolo[1,5-c]pyrimidin-Verbindung der Formel

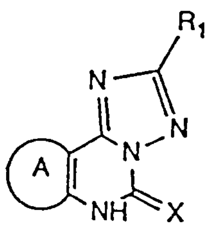

(I),

worin a) X Sauerstoff, $R_1$ 2-Fluorphenyl und der Ring A $\Delta^3$-Piperidein, an Stickstoff substituiert durch Methoxycarbonylmethyl, 3-Picolyl, 2-Picolyl, 4-Picolyl, Phenylsulfonyl, Carboxymethyl, Carbamoylmethyl, Carbamoyl, Cyanomethyl, Tetrazolylmethyl, (+)-α-Methylbenzyl oder (-)-α-Methylbenzyl, ist; oder b) X Sauerstoff, $R_1$ 2-Chlorophenyl, 2-Pyridyl oder 2-Pyrrolyl und Ring A N-Benzyl-$\Delta^3$-piperidein ist; oder c) X Imino, $R_1$ 2-Furyl und der Ring A Cyclopenten ist; ein Tautomer davon oder ein pharmazeutisch annehmbares Salz der Verbindung oder des Tautomers.

2. Verbindung nach Anspruch 1 der Formel I, worin X Sauerstoff, $R_1$ 2-Chlorphenyl und der Ring A N-Benzyl-$\Delta^3$-piperidein ist, mit dem Namen 9-Benzyl-2-(2-chlorphenyl)-7,8,9,10-tetrahydropyrido[3,4-e]-[1,2,4]triazolo[1,5-c]-pyrimidin-5(6H)on; ein Tautomer davon oder ein pharmazeutisch annehmbares Salz davon.

3. Verbindung nach Anspruch 1 mit der Formel I, worin X Sauerstoff, $R_1$ 2-Pyridyl und der Ring A N-Benzyl-$\Delta^3$-piperidein ist, mit dem Namen 9-Benzyl-2-(2-pyridyl)-7,8,9,10-tetrahydropyrido[3,4-e][1,2,4]-triazolo[1,5-c]-pyrimidin-5(6H)on; ein Tautomer davon oder ein pharmazeutisch annehmbares Salz davon.

4. Verbindung nach Anspruch 1 der Formel I, worin X Sauerstoff, $R_1$ 2-Pyrrolyl und der Ring A N-Benzyl-$\Delta^3$-piperidein ist, mit dem Namen 9-Benzyl-2-(2-pyrrolyl)-7,8,9,10-tetrahydropyrido[3,4-e][1,2,4]triazolo-[1,5-c]pyrimidin-5(6H)on; ein Tautomer davon oder ein pharmazeutisch annehmbares Salz davon.

5. Verbindung nach Anspruch 1 der Formel I, worin X Imino, $R_1$ 2-Furyl und der Ring A N-Benzvl-$\Delta^3$-piperidein ist, mit dem Namen 2-(2-Furyl)-5-imino-5,6,8,9-tetrahydro-7H-cyclopenta[e][1,2,4]triazolo[1,5-c]pyrimidin; ein Tautomer davon oder ein pharmazeutisch annehmbares Salz davon.

6. Verbindung nach Anspruch 1 der Formel I, worin X Sauerstoff, $R_1$ 2-Fluorphenyl und der Ring A N-(Methoxycarbonylmethyl)-$\Delta^3$-piperidein ist, mit dem Namen 9-Carbomethoxy-methyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon.

7. Verbindung nach Anspruch 1 der Formel I, worin X Sauerstoff, $R_1$ 2-Fluorphenyl und der Ring A N-(3-Picolyl)-$\Delta^3$-piperidein ist, mit dem Namen 2-(2-Fluorphenyl)-9-(3-picolyl)-7,8,-9,10-tetrahydro-pyrido[3,4-e][1,2,4]triazolo[1,5-c]pyrimidin-5(6H)on; ein Tautomer davon oder ein pharmazeutisch annehmbares Salz davon.

8. Verbindung nach Anspruch 1 der Formel I, worin X Sauerstoff, $R_1$ 2-Fluorphenyl und der Ring A N-(2- oder 4-Picolyl)-$\Delta^3$-piperidein ist, mit dem Namen 2-(2-Fluorphenyl)-9-(2-picolyl)-7,8,9,10-tetrahydro-pyrido[3,4-e][1,2,4]triazolo[1,5-c]pyrimidin-5(6H)on oder 2-(2-Fluorphenyl)-9-(4-picolyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**9.** Verbindung nach Anspruch 1 der Formel I, worin X Sauerstoff, $R_1$ 2-Fluorphenyl und der Ring A N-Phenylsulfonyl-$\Delta^3$-piperidein ist, mit dem Namen 2-(2-Fluorphenyl)-9-phenylsulfonyl-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**10.** Verbindung nach Anspruch 1 der Formel I, worin X Sauerstoff, $R_1$ 2-Fluorphenyl und der Ring A N-Carboxymethyl-$\Delta^3$-piperidein ist, mit dem Namen 9-Carboxymethyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**11.** Verbindung nach Anspruch 1 der Formel I, worin X Sauerstoff, $R_1$ 2-Fluorphenyl und der Ring A N-(Carbamoylmethyl)-$\Delta^3$-piperidein ist, mit dem Namen 9-Carbamoylmethyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**12.** Verbindung nach Anspruch 1 der Formel I, worin X Sauerstoff, $R_1$ 2-Fluorphenyl und der Ring A N-(Carbamoyl)-$\Delta^3$-piperidein ist, mit dem Namen 9-Carbamoyl-2-(2-fluorphenyl)-7,8,9,10-tetrahydropyrido-[3,4-e][1,2,4]triazolo[1,5-c]pyrimidin-5(6H)on; ein Tautomer davon oder ein pharmazeutisch annehmbares Salz davon.

**13.** Verbindung nach Anspruch 1 der Formel I, worin X Sauerstoff, $R_1$ 2-Fluorphenyl und der Ring A N-Cyanomethyl-$\Delta^3$-piperidein ist, mit dem Namen 9-Cyanomethyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**14.** Verbindung nach Anspruch 1 der Formel I, worin X Sauerstoff, $R_1$ 2-Fluorphenyl und der Ring A N-(Tetrazolylmethyl)-$\Delta^3$-piperidein ist, mit dem Namen 2-(2-Fluorphenyl)-9-tetrazolylmethyl-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**15.** Verbindung nach Anspruch 1 der Formel I, worin X Sauerstoff, $R_1$ 2-Fluorphenyl und der Ring A N-((+)- oder (-)-$\alpha$-Methylbenzyl)-$\Delta^3$-piperidein ist, mit dem Namen (+)- oder (-)-2-(2-Fluor)-9-$\alpha$-methylbenzyl-7,8,9,10-tetrahydropyrido[3,4-e][1,2,4]triazolo[1,5-c]pyrimidin-5(6H)on; ein Tautomer davon oder ein pharmazeutisch annehmbares Salz davon.

**16.** Verbindungen nach den Ansprüchen 1 bis 15 zur Verwendung bei der Behandlung von Angst oder Asthma oder als Adenosin-Antagonisten.

**17.** Pharmazeutische Zusammensetzungen, die eine wirksame Menge einer Verbindung nach den Ansprüchen 1 - 15 und einen pharmazeutisch annehmbaren Träger enthalten.

**18.** Verfahren zur Herstellung einer Verbindung der Formel I, worin X, $R_1$ und A wie im Anspruch 1 definiert sind, umfassend: a) Cyclisierung einer Verbindung der Formel

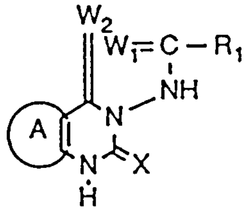

(II),

worin $R_1$, X und der Ring A wie oben definiert sind, eines von $W_1$ und $W_2$ NH ist und das andere von $W_1$ und $W_2$ O oder NH darstellt, durch Behandlung mit einer Base, die ein Stickstoffatom liefert, oder b) um eine Verbindung der Formel I zu erhalten, worin X Sauerstoff ist, Cyclisierung einer Verbindung der Formel:

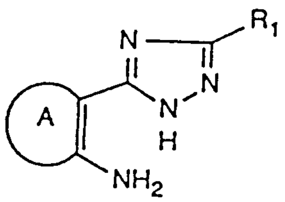

(III)

worin $R_1$ und der Ring A wie oben definiert sind, durch Behandlung mit einem reaktiven Derivat von Kohlensäure, oder c) um eine Verbindung der Formel I zu erhalten, in der X Sauerstoff ist, Umsetzung einer Verbindung der Formel:

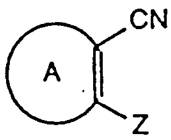

(IV)

worin der Ring A wie oben definiert ist und Z ein Rest eines Kohlensäurederivats ist, der über ein Stickstoffatom gebunden ist, mit einem Hydrazid der Formel:

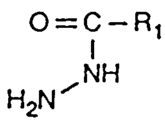

(V)

d) um eine Verbindung der Formel I zu erhalten, worin X Sauerstoff ist, Behandlung einer Verbindung der Formel:

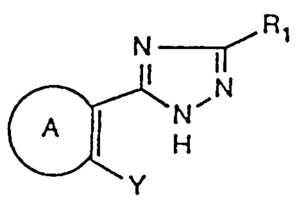

(VI)

worin Y eine Gruppe darstellt, die befähigt ist, in die Gruppierung -N=C=O durch ein Oxidationsmittel umgewandelt zu werden, mit besagtem Oxidationsmittel gefolgt von Ringschluß, oder e) um eine Verbindung der Formel I zu erhalten, worin X NH ist, Umwandeln einer Verbindung der Formel:

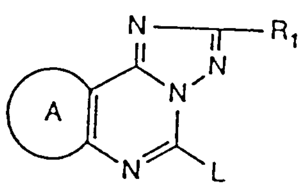

(VII)

worin $R_1$ 2-Furyl ist, der Ring A Cyclopentan ist und L ausgewählt wird aus Halogen, $C_1$-$C_7$-Alkoxy, Aryl-$C_1$-$C_7$-Alkoxy, Mercapto, $C_1$-$C_7$-Alkylthio und Isothiocyanato, durch Ersetzen der Gruppe L durch die Gruppierung NH, oder f) Behandlung einer Verbindung der Formel:

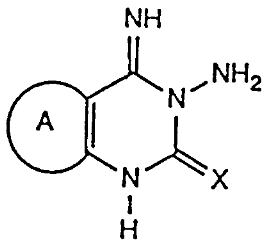

(VIII)

mit einem reaktiven Derivat einer Carbonsäure der Formel $R_1$-COOH, oder g) um eine Verbindung der Formel I zu erhalten, worin X Imino ist, Behandlung einer Verbindung der Formel I, worin X NR ist, worin R $C_1$-$C_7$-Alkyl-, $C_2$-$C_7$-Alkenyl oder $C_2$-$C_7$-Alkinyl ist, mit wäßriger Säure, oder h) um eine Verbindung der Formel I zu erhalten, worin X NH ist, i) Umsetzung einer Verbindung der Formel III, wie im Verfahren (b) oben definiert, mit Cyanhalogenid gefolgt von Cyclisierung oder Umsetzung einer Verbindung der Formel III mit Cyanamid oder einem reaktiven Iminocarbonylderivat, oder i) um eine Verbindung der Formel I zu erhalten, worin X Sauerstoff ist, Umsetzung einer Verbindung der Formel

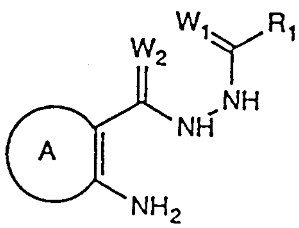

(IX)

miteinem reaktiven Derivat von Kohlensäure und Base, oder j) um eine Verbindung der Formel I zu erhalten, worin X Sauerstoff ist, Hydrolysieren einer Verbindung der Formel

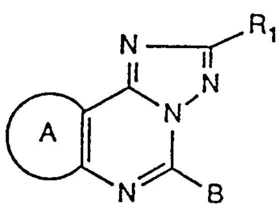

XV

worin B Halo oder $C_1$-$C_7$-Alkoxy ist, oder k) um die Verbindung der Formel I zu erhalten, worin X Imino ist, durch doppeltes Cyclisieren einer Verbindung der Formel

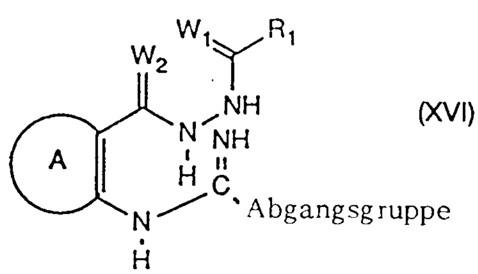

(XVI)

oder der Formel

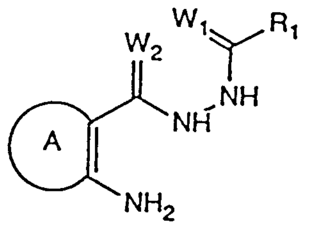

(XVII)

oder der Formel

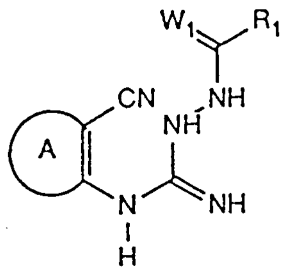

(XVIII),

wobei die Reaktion der Verbindung der Formel XVII in Gegenwart von einem Cyanhalogenid oder Cyanamid oder einem reaktiven Iminokohlensäurederivat erfolgt, oder l) um eine Verbindung der Formel I zu erhalten, worin X Imino ist, Cyclisierung einer Verbindung der Formel

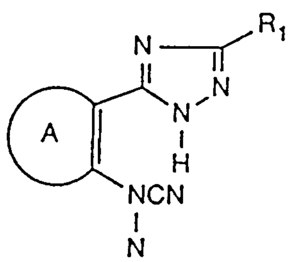

XX

die aus einer Verbindung der Formel III durch Umsetzung davon mit Cyanhalogenid hergestellt wird, oder m) um eine Verbindung der Formel I zu erhalten, worin X Imino ist, Umsetzung einer Verbindung der Formel

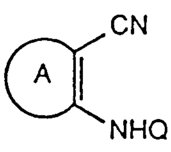

XXI

worin Q für-CN oder Formel

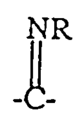

Abgangsgruppe steht, mit einem Hydrazid der Forme V;
und, falls gewünscht, Umwandlung einer resultierenden Verbindung in eine andere erfindungsgemäße Verbindung und/oder Umwandlung eines resultierenden Salzes in die freie Verbindung oder in ein anderes Salz und/oder Umwandlung einer resultierenden freien Verbindung, die eine salzbildende

Gruppe hat, in ein Salz.

**Patentansprüche für folgende Vertragsstaaten : ES, GR**

1. Verfahren zur Herstellung einer Verbindung der Formel

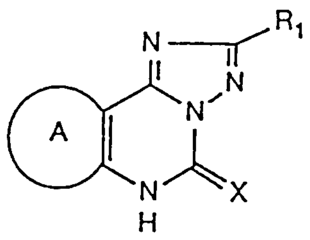

(I),

worin a) X Sauerstoff, $R_1$ 2-Fluorphenyl und der Ring A $\Delta^3$-Piperidein, an Stickstoff substituiert durch Methoxycarbonylmethyl, 3-Picolyl, 2-Picolyl, 4-Picolyl, Phenylsulfonyl, Carboxymethyl, Carbamoylmethyl, Carbamoyl, Cyanomethyl, Tetrazolylmethyl, (+)-$\alpha$-Methylbenzyl oder (-)-$\alpha$-Methylbenzyl, ist; oder b) X Sauerstoff, $R_1$ 2-Chlorphenyl, 2-Pyridyl oder 2-Pyrrolyl und Ring A N-Benzyl-$\Delta^3$-piperidein ist; oder c) X Imino, $R_1$ 2-Furyl und der Ring A Cyclopenten ist; eines Tautomers davon oder eines pharmazeutisch annehmbaren Salzes der Verbindung oder des Tautomers, welches Verfahren umfaßt: a) Cyclisierung einer Verbindung der Formel

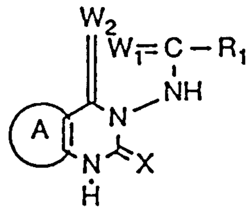

(II),

worin $R_1$, X und der Ring A wie oben definiert sind, eines von $W_1$ und $W_2$ NH ist und das andere von $W_1$ und $W_2$ O oder NH darstellt, durch Behandlung mit einer Base, die ein Stickstoffatom liefert, oder b) um eine Verbindung der Formel I zu erhalten, worin X Sauerstoff ist, Cyclisierung einer Verbindung der Formel:

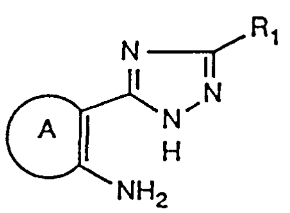

(III)

worin $R_1$ und der Ring A wie oben definiert sind, durch Behandlung mit einem reaktiven Derivat von Kohlensäure, oder c) um eine Verbindung der Formel I zu erhalten, in der X Sauerstoff ist, Umsetzung einer Verbindung der Formel:

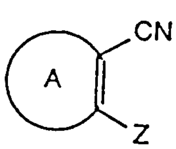

(IV)

worin der Ring A wie oben definiert ist und Z ein Resteines Kohlensäurederivats ist, der über ein Stickstoffatom gebunden ist, mit einem Hydrazid der Formel:

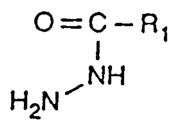

(V)

d) um eine Verbindung der Formel I zu erhalten, worin X Sauerstoff ist, Behandlung einer Verbindung der Formel:

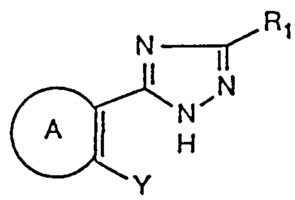

(VI)

worin Y eine Gruppe darstellt, die befähigt ist, in die Gruppierung -N = C = O durch ein Oxidationsmittel umgewandelt zu werden, mit besagtem Oxidationsmittel gefolgt von Ringschluß, oder e) um eine Verbindung der Formel I zu erhalten, worin X NH ist, Umwandeln einer Verbindung der Formel:

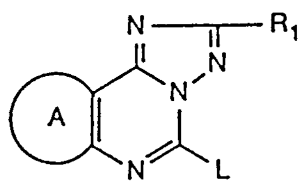

(VII)

worin $R_1$ 2-Furyl ist, der Ring A Cyclopentan ist und L ausgewählt wird aus Halogen, $C_1$-$C_7$-Alkoxy, Aryl-$C_1$-$C_7$-Alkoxy, Mercapto, $C_1$-$C_7$-Alkylthio und Isothiocyanato, durch Ersetzen der Gruppe L durch die Gruppierung NH, oder f) Behandlung einer Verbindung der Formel:

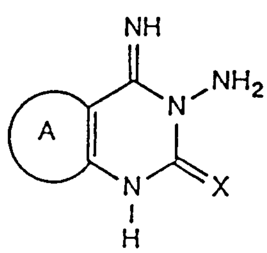

(VIII)

mit einem reaktiven Derivat einer Carbonsäure der Formel $R_1$-COOH, oder g) um eine Verbindung der Formel I zu erhalten, worin X Imino ist, Behandlung einer Verbindung der Formel I, worin X NR ist, worin R $C_1$-$C_7$-Alkyl-, $C_2$-$C_7$-Alkenyl oder $C_2$-$C_7$-Alkinyl ist, mit wäßriger Säure, oder h) um eine Verbindung der Formel I zu erhalten, worin X NH ist, i) Umsetzung einer Verbindung der Formel III, wie im Verfahren (b) oben definiert, mit Cyanhalogenid gefolgt von Cyclisierung oder Umsetzung einer Verbindung der Formel III mit Cyanamid oder einem reaktiven Iminocarbonylderivat, oder i) um eine Verbindung der Formel I zu erhalten, worin X Sauerstoff ist, Umsetzung einer Verbindung der Formel

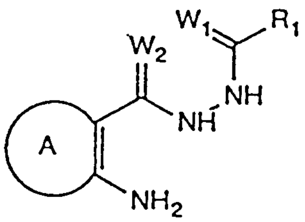

(IX)

miteinem reaktiven Derivat von Kohlensäure und Base, oder j) um eine Verbindung der Formel I zu erhalten, worin X Sauerstoff ist, Hydrolysieren einer Verbindung der Formel

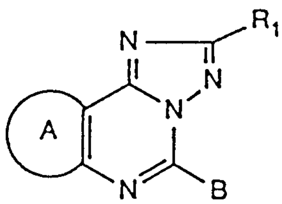

XV

worin B Halo oder $C_1$-$C_7$-Alkoxy ist, oder k) um die Verbindung der Formel I zu erhalten, worin X Imino ist, durch doppeltes Cyclisieren einer Verbindung der Formel

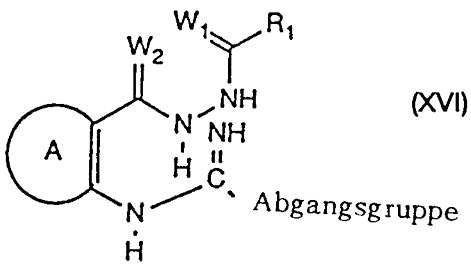

(XVI)

oder der Formel

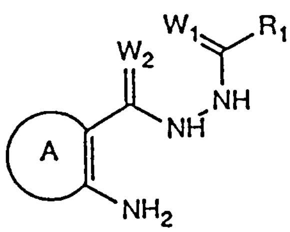

(XVII)

oder der Formel

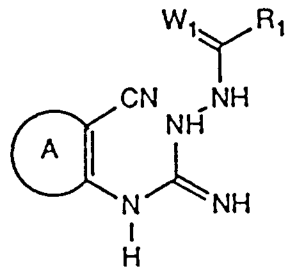

(XVIII),

wobei die Reaktion der Verbindung der Formel XVII in Gegenwart von einem Cyanhalogenid oder Cyanamid oder einem reaktiven Iminokohlensäurederivat erfolgt, oder l) um eine Verbindung der Formel I zu erhalten, worin X Imino ist, Cyclisierung einer Verbindung der Formel

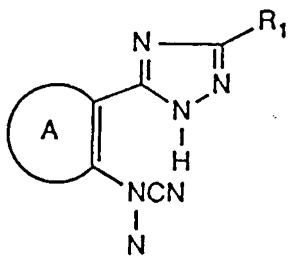

XX

die aus einer Verbindung der Formel III durch Umsetzung davon mit Cyanhalogenid hergestellt wird, oder m) um eine Verbindung der Formel I zu erhalten, worin X Imino ist, Umsetzung einer Verbindung der Formel

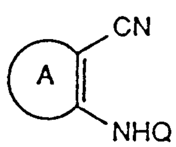

XXI

worin Q für -CN oder Formel

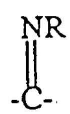

Abgangsgruppe steht, mit einem Hydrazid der Formel V;
und, falls gewünscht, Umwandlung einer resultierenden Verbindung in eine andere erfindungsgemäße Verbindung und/oder Umwandlung eines resultierenden Salzes in die freie Verbindung oder in ein anderes Salz und/oder Umwandlung einer resultierenden freien Verbindung, die eine salzbildende Gruppe hat, in ein Salz.

**2.** Verfahren nach Anspruch 1, worin die Ausgangsmaterialien so gewählt werden, daß die Verbindung der Formel I, worin X Sauerstoff, $R_1$ 2-Chlorphenyl und der Ring A N-Benzyl-$\Delta^3$-piperidein ist, mit dem Namen 9-Benzyl-2-(2-chlorphenyl)-7,8,9,10-tetrahydropyrido[3,4-e][1,2,4]triazolo[1,5-c]-pyrimidin-5(6H)-on; ein Tautomer davon oder ein pharmazeutisch annehmbares Salz davon hergestellt wird.

**3.** Verfahren nach Anspruch 1, worin die Ausgangsmaterialien so gewählt werden, daß die Verbindung der Formel I, worin X Sauerstoff, $R_1$ 2-Pyridyl und der Ring A N-Benzyl-$\Delta^3$-piperidein ist, mit dem Namen 9-Benzyl-2-(2-pyridyl)-7,8,9,10-tetrahydropyrido[3,4-e][1,2-e]triazolo[1,5-c]-pyrimidin-5(6H)on; ein Tautomer davon oder ein pharmazeutisch annehmbares Salz davon hergestellt wird.

**4.** Verfahren nach Anspruch 1, worin die Ausgangsmaterialien so gewählt werden, daß die Verbindung der

Formel I, worin X Sauerstoff, $R_1$ 2-Pyrrolyl und der Ring A N-Benzyl-$\Delta^3$-piperidein ist, mit dem Namen 9-Benzyl-2-(2-pyrrolyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.

**5.** Verfahren nach Anspruch 1, worin die Ausgangsmaterialien so gewählt werden, daß die Verbindung der Formel I, worin X Imino, $R_1$ 2-Furyl und der Ring A N-Benzyl-$\Delta^3$-piperidein ist, mit dem Namen 2-(2-Furyl)-5-imino-5,6,8,9-tetrahydro-7H-cyclopenta[e][1,2,4]triazolo[1,5-c]pyrimidin; ein Tautomer davon oder ein pharmazeutisch annehmbares Salz davon hergestellt wird.

**6.** Verfahren nach Anspruch 1, worin die Ausgangsmaterialien so gewählt werden, daß die Verbindung der Formel I, worin X Sauerstoff, $R_1$ 2-Fluorphenyl und der Ring A N-(Methoxycarbonylmethyl)-$\Delta^3$-piperidein ist, mit dem Namen 9-Carbomethoxy-methyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.

**7.** Verfahren nach Anspruch 1, worin die Ausgangsmaterialien so gewählt werden, daß die Verbindung der Formel I, worin X Sauerstoff, $R_1$ 2-Fluorphenyl und der Ring A N-(3-Picolyl)-$\Delta^3$-piperidein ist, mit dem Namen 2-(2-Fluorphenyl)-9-(3-picolyl)-7,8,9,10-tetrahydro-pyrido[3,4-e][1,2,4]triazolo[1,5-c]pyrimidin-5-(6H)on; ein Tautomer davon oder ein pharmazeutisch annehmbares Salz davon hergestellt wird.

**8.** Verfahren nach Anspruch 1, worin die Ausgangsmaterialien so gewählt werden, daß die Verbindung der Formel I, worin X Sauerstoff, $R_1$ 2-Fluorphenyl und der Ring A N-(2- oder 4-Picolyl)-$\Delta^3$-piperidein ist, mit dem Namen 2-(2-Fluorphenyl)-9-(2-picolyl)-7,8,9,10-tetrahydro-pyrido[3,4-e][1,2,4]triazolo[1,5-c]-pyrimidin-5(6H)on oder 2-(2-Fluorphenyl)-9-(4-picolyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.

**9.** Verfahren nach Anspruch 1, worin die Ausgangsmaterialien so gewählt werden, daß die Verbindung der Formel I, worin X Sauerstoff, $R_1$ 2-Fluorphenyl und der Ring A N-Phenylsulfonyl-$\Delta^3$-piperidein ist, mit dem Namen 2-(2-Fluorphenyl)-9-phenylsulfonyl-7,8,9,10-tetrahydropyrido-[3,4-e][1,2,4]triazolo[1,5-c]-pyrimidin-5(6H)on; ein Tautomer davon oder ein pharmazeutisch annehmbares Salz davon hergestellt wird.

**10.** Verfahren nach Anspruch 1, worin die Ausgangsmaterialien so gewählt werden, daß die Verbindung der Formel I, worin X Sauerstoff, $R_1$ 2-Fluorphenyl und der Ring A N-Carboxymethyl-$\Delta^3$-piperidein ist, mit dem Namen 9-Carboxymethyl-2-(2-fluorphenyl)-7,8,9,10-tetrahydropyrido [3,4-e][1,2,4]triazolo[1,5-c]-pyrimidin-5(6H)on; ein Tautomer davon oder ein pharmazeutisch annehmbares Salz davon hergestellt wird.

**11.** Verfahren nach Anspruch 1, worin die Ausgangsmaterialien so gewählt werden, daß die Verbindung der Formel I, worin X Sauerstoff, $R_1$ 2-Fluorphenyl und der Ring A N-(Carbamoylmethyl)-$\Delta^3$-piperidein ist, mit dem Namen 9-Carbamoylmethyl-2-(2-fluorphenyl)-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.

**12.** Verfahren nach Anspruch 1, worin die Ausgangsmaterialien so gewählt werden, daß die Verbindung der Formel I, worin X Sauerstoff, $R_1$ 2-Fluorphenyl und der Ring A N-(Carbamoyl)-$\Delta^3$-piperidein ist, mit dem Namen 9-Carbamoyl-2-(2-fluorphenyl)-7,8,9,10-tetrahydropyrido [3,4-e][1,2,4]triazolo[1,5-c]-pyrimidin-5(6H)on; ein Tautomer davon oder ein pharmazeutisch annehmbares Salz davon hergestellt wird.

**13.** Verfahren nach Anspruch 1, worin die Ausgangsmaterialien so gewählt werden, daß die Verbindung der Formel I, worin X Sauerstoff, $R_1$ 2-Fluorphenyl und der Ring A N-Cyanomethyl-$\Delta^3$-piperidein ist, mit dem Namen 9-Cyanomethyl-2-(2-fluorphenyl)-7,8,9,10-tetrahydropyrido [3,4-e][1,2,4]triazolo[1,5-c]-pyrimidin-5(6H)on; ein Tautomer davon oder ein pharmazeutisch annehmbares Salz davon hergestellt wird.

**14.** Verfahren nach Anspruch 1, worin die Ausgangsmaterialien so gewählt werden, daß die Verbindung der

Formel I, worin X Sauerstoff, $R_1$ 2-Fluorphenyl und der Ring A N-(Tetrazolylmethyl)-$\Delta^3$-piperidein ist, mit dem Namen 2-(2-Fluorphenyl)-9-tetrazolylmethyl-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.

**15.** Verfahren nach Anspruch 1, worin die Ausgangsmaterialien so gewählt werden, daß die Verbindung der Formel I, worin X Sauerstoff, $R_1$ 2-Fluorphenyl und der Ring A N-((+)- oder (-)-α-Methylbenzyl)-$\Delta^3$-piperidein ist, mit dem Namen (+)- oder (-)-2-(2-Fluor)-9-α-methylbenzyl-7,8,9,10-tetrahydropyrido[3,4-e][1,2,4]triazolo[1,5-c]pyrimidin-5(6H)on; ein Tautomer davon oder ein pharmazeutisch annehmbares Salz davon hergestellt wird.